(19) Europäisches Patentamt
European Patent Office
Office européen des brevets

(11) EP 2 570 127 A1

(12) EUROPEAN PATENT APPLICATION

(43) Date of publication:
**20.03.2013 Bulletin 2013/12**

(21) Application number: **11306172.5**

(22) Date of filing: **16.09.2011**

(51) Int Cl.:
***A61K 31/4184*** *(2006.01)* ***A61K 31/437*** *(2006.01)*
***A61K 31/5377*** *(2006.01)* ***A61K 45/06*** *(2006.01)*
***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventor: **The designation of the inventor has not yet been filed**

(74) Representative: **Blot, Philippe Robert Emile**
**Cabinet Lavoix**
**2, place d'Estienne d'Orves**
**75441 Paris Cedex 09 (FR)**

(54) **Compositions and methods for treating cancer using PI3KB beta inhibitor and MAPK pathway inhibitor, including MEK and RAF inhibitors**

(57) The present invention relates to compositions comprising at least one MAPK pathway inhibitor including MEK and RAF inhibitors and at least one PI3Kβ inhibitor, and also to uses thereof for the treatment of cancer.



Printed by Jouve, 75001 PARIS (FR)

## Description

There is an ongoing need in the art for more efficacious methods and compositions in the treatment of cancer. The present invention concerns generally, compositions and uses thereof for the treatment of cancer, and more particularly, compositions comprising inhibitors of phosphoinositide 3-kinaseβ (PI3Kβ or PI3K beta) and inhibitors of MAPK (Mitogen Activated Protein Kinase) pathways, including the MEK (Mitogen-activated protein kinase, also known as MAP2K) and RAF kinase inhibitors.

Phosphoinositide 3-kinases (PI3Ks) are signaling molecules involved in numerous cellular functions such as cell cycle, cell motility and apoptosis. PI3Ks are lipid kinases that produce second messenger molecules activating several target proteins including serine/threonine kinases like PDK1 and AKT (also known as PKB). PI3Ks are divided in three classes and class I comprises four different PI3Ks named PI3K alpha, PI3K beta, PI3K delta and PI3K gamma.

PI3Kβ is a class IA member that is ubiquitously expressed and possesses the unique feature of being activated not only by tyrosine kinase receptors, but also by G protein-coupled receptors (Vanhaesebroeck et al., 2001).

2-{2-[(2S)-2-methyl-2,3-dihydro-1H-indol-1-yl]-2-oxoethyl}-6-(morpholin-4-yl)pyrimidin-4(3H)-one is a selective inhibitor of the P13Kβ isoform of the class I phosphoinositide-3 kinase (PI3K) lipid kinase. This compound potently targets PI3Kβ isoform with an IC50 of 65 nM and is selective versus other PI3K isoforms with an IC50 of 1188 nM, 465 nM and > 10 000 nM on PI3K alpha, PI3K delta and PI3K gamma, respectively. It inhibits the phosphorylation and activation of Akt as well as Akt downstream effectors.

After treatment with this compound, tumor cells with an activated PI3K/AKT pathway, as for example PTEN-deficient tumor cells, typically respond *via* inhibition of phosphorylation of Akt as well as of Akt downstream effectors, inhibition of tumor cell proliferation and tumor cell death induction.

Tumor cells treated with inhibitors of MEK kinases typically respond *via* inhibition of phosphorylation of ERK (extracellular-signal-regulated kinase), down-regulation of Cyclin D1, induction of G1 arrest, and finally undergo apoptosis. Pharmacologically, MEK inhibition completely abrogates tumor growth in BRAF mutant xenograft tumors whereas Ras mutant tumors exhibit only partial inhibition in most cases (D. B. Solit et al., Nature 2006; 439: 358-362). Thus, MEKs have been targets of great interest for the development of cancer therapeutics.

Tumor cells treated with inhibitors of RAF kinase typically respond via inhibition of phosphorylation of MEK and of ERK, down-regulation of Cyclin D, induction of G1 arrest, and finally undergo apoptosis. Pharmacologically, BRAF-V600E inhibition completely abrogates tumor growth in *BRAF* mutant xenograft tumors. Thus, RAFs have been targets of great interest for the development of cancer therapeutics.

1 H-Benzimidazole-6-carboxamide, 5-[(4-bromo-2-chlorophenyl)amino]-4-fluoro-N-(2-hydroxyethoxy)-1-methyl (also referred as AZD-6244 or Selumetinib) is an allosteric inhibitor of MEK kinase with high potency and selectivity *versus* other kinases. Selumetinib is an oral MEK1/2 inhibitor, for the potential treatment of solid tumors as non-small-cell lung cancer (NSCLC), pancreatic cancer, colorectal cancer, biliary cancer, thyroid carcinoma, and malignant melanoma.

1-Propanesulfonamide, N-[3-[[5-(4-chlorophenyl)-l H-pyrrolo[2,3-b]pyridin-3-yl]carbonyl]-2,4-difluorophenyl] (also referred as PLX 4032 or Vemurafenib) is an inhibitor of RAF kinases. It inhibits the activity of BRAF (V600E), wild-type BRAF and CRAF-1 with IC50s of 31, 100 and 48 nM, respectively. It displays selectivity *versus* many other kinases. PLX-4032 is an orally available small-molecule, developed for the treatment of cancers harboring activating BRAF mutations. It has marked antitumor effects against melanoma cell lines with the BRAF V600E mutation but not against cells with wild-type BRAF.

There remains a need, for a cancer therapy that is more effective in inhibiting cell proliferation and tumor growth while minimizing patient toxicity. There is a particular need for a MEK or RAF inhibitor therapy used in combination with other targeted therapy leading to more efficiency without substantially increasing, or even maintaining or decreasing, the dosages of MEK, or RAF inhibitor traditionally employed in the art.

In particular, the instant application is directed to combination of a PI3Kβ selective inhibitor with a modulator of the MAPK pathway, including MEK and RAF inhibitors.

In particular, the instant application is directed to combination of a PI3Kβ selective inhibitor with a MEK inhibitor or a RAF inhibitor.

In particular, the instant application is directed to combination of a PI3Kβ selective inhibitor with a MEK inhibitor.

In particular, the instant application is directed to combination of a PI3Kβ selective inhibitor with a RAF inhibitor.

Accordingly, the present invention relates to a pharmaceutical combination comprising:

- at least one compound of formula (I):

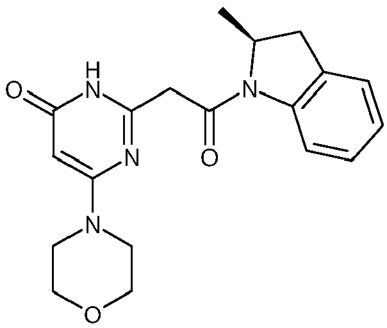

(I)

or a pharmaceutically acceptable salt thereof,
and

- at least one MAPK pathway inhibitor.

According to an embodiment, in the pharmaceutical combination of the invention, the MAPK pathway inhibitor is chosen from the group consisting of the inhibitors of MEK and RAF kinases.

According to an embodiment, in the pharmaceutical combination of the invention, the MAPK pathway inhibitor is an inhibitor of one or both of a MEK kinase and a RAF kinase.

The present invention also relates to a pharmaceutical combination as defined above, wherein the MAPK pathway inhibitor is a MEK inhibitor.

According to an embodiment, the MAPK pathway inhibitor is a RAF inhibitor. According to an embodiment, the MAPK pathway inhibitor is a BRAF inhibitor.

According to an embodiment, the compound of formula (I) as defined above is a PI3K inhibitor, in particular a PI3Kβ inhibitor.

In one aspect, there is provided compositions and uses thereof in the treatment of a variety of cancers.

In particular embodiments, there is provided a composition that includes a MAPK pathway inhibitor, including MEK and RAF inhibitors, and a compound having the following structural formula (I) as defined above.

In a particular embodiment , there is provided a composition that includes a MAPK pathway inhibitor, including MEK and RAF inhibitors, and a PI3Kβ inhibitor, such inhibitor of PI3Kβ having the above-mentioned structural formula (I).

In particular embodiments, there is provided a composition that includes a MEK inhibitor or a RAF inhibitor and a PI3Kβ inhibitor, such inhibitor of PI3Kβ having formula (I) as defined above.

In particular embodiments, there is provided a composition that includes a MEK inhibitor and a PI3Kβ inhibitor, such inhibitor of PI3Kβ having the formula (I) as defined above.

In particular embodiments, there is provided a composition that includes a RAF inhibitor and a PI3Kβ inhibitor, such inhibitor of PI3Kβ having the formula (I) as defined above.

In particular embodiments, there is provided a composition that includes a BRAF inhibitor and a PI3Kβ inhibitor, such inhibitor of PI3Kβ having the formula (I) as defined above.

In the above compositions, such MAPK pathway inhibitors, including MEK and RAF inhibitors, may be chosen among the inhibitors known by the man of the art and then may be chosen for example among:

i) MEK inhibitors: AZD6244, RO4987655, RO5126766, TAK-733, MSC1936369B (AS703026), GSK1120212, BAY86-9766, GDC-0973, GDC-0623, PD325901, ARRY-438162, CI1040, E6201, ARRY300

ii) RAF and/or BRAF selective inhibitors: PLX4032, GSK2118436, Sorafenib (BAY-43-9006), BMS-908662 (XL-281), RAF265, RG-7256 (RO5212054, PLX3603), RO5126766, ARQ-736, E-3810, DCC-2036.

According to a specific embodiment, in the pharmaceutical combination of the invention, the MAPK pathway inhibitor is chosen from the group consisting of:

- the compound (2a):

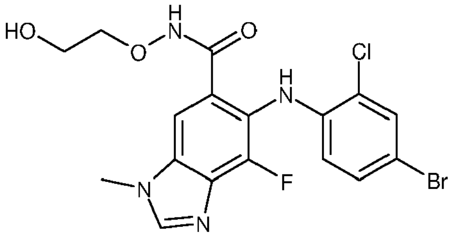

(2a)

or a pharmaceutically acceptable salt thereof, and

- the compound (2b):

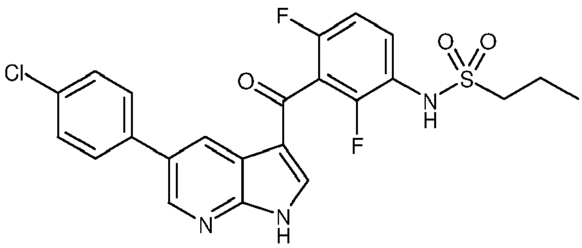

(2b)

or a pharmaceutically acceptable salt thereof.

In particular embodiments, there is provided a composition that includes a compound having the above formula (I) and a compound of the above formula (2a).

In particular embodiments, there is provided a composition that includes a compound having the above formula (I)and a compound of the above formula (2b).In particular embodiments, there is provided a composition that includes a PI3Kβ inhibitor having the above formula (I) and a MEK inhibitor having the above formula (2a).

In particular embodiments, there is provided a composition that includes a PI3Kβ inhibitor having the above formula (I) and a RAF inhibitor having the above formula (2b).

The present invention also relates to a pharmaceutical combination as defined above, wherein the MAPK pathway inhibitor is the compound (2a) of formula:

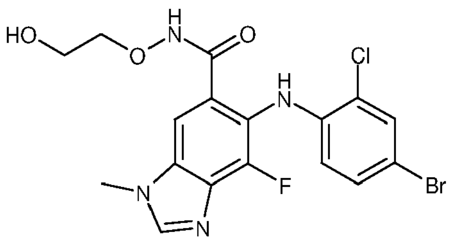

or a pharmaceutically acceptable salt thereof.

The present invention also relates to a pharmaceutical combination as defined above, wherein the MAPK pathway inhibitor is the compound (2b) of formula:

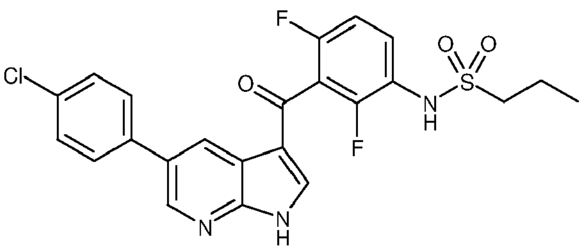

or a pharmaceutically acceptable salt thereof.

According to an embodiment, the pharmaceutical combination of the invention may further comprise a pharmaceutically acceptable carrier.

According to an embodiment, the pharmaceutical combination of the invention may comprise at least one further compound chosen from anticancer compounds.

According to an embodiment, in the pharmaceutical combination of the invention, Compound (I) can be administered at a dosage that will allow PI3Kβ target inhibition in human tumors and that will be dosages anticipated to be of about 60-600 mg po bid or - 120-1200 mg po qd.

According to an embodiment, in the pharmaceutical combination of the invention, the amount of the MAPK pathway inhibitor may be from 10 mg/kg to 200 mg/kg qd or bid.

According to an embodiment, in the pharmaceutical combination of the invention, the amount of the MEK inhibitor can be administered at a dosage of about 2-200 mg qd or bid po.

According to an embodiment, in the pharmaceutical combination of the invention, the RAF inhibitor can be administered at a dosage of about 60-200 mg bid po.

According to an embodiment, in the pharmaceutical combination of the invention, the compound (2a) inhibitor be administered at a dosage of about 2-200 mg qd or bid po .

According to an embodiment, in the pharmaceutical combination of the invention, the compound (2b) inhibitor be administered at a dosage of about 60-200 mg bid po.

The present invention also relates to a medicament comprising the pharmaceutical combination as defined above.

The present invention also relates to a pharmaceutical composition comprising the pharmaceutical combination as defined above, and a pharmaceutically acceptable excipient.

The present invention also relates to a pharmaceutical combination as defined above, for its use as a medicament.

The present invention also relates to a pharmaceutical combination as defined above, for its use for the treatment of cancer.

According to an embodiment, the cancer is chosen from the group consisting of: non-small cell lung cancer, breast cancer, pancreatic cancer, liver cancer, prostate cancer, bladder cancer, cervical cancer, thyroid cancer, colorectal cancer, liver cancer, muscle cancer, hematological malignancies, melanoma, endometrial cancer and pancreatic cancer.

According to an embodiment, the cancer is chosen from the group consisting of any colorectal cancer, endometrial cancer, hematological malignancies, thyroid cancer, breast cancer, melanoma, pancreatic cancer and prostate cancer.

According to an embodiment, the present invention relates to the pharmaceutical combination as defined above for its use for the treatment of cancer, by administration of the compound of formula (I) and the administration of the MAPK pathway inhibitor.

According to an embodiment, the present invention relates to the pharmaceutical combination as defined above for its use for the treatment of cancer, by administration of the compound of formula (I) and the administration of the compound (2a).

According to an embodiment, the present invention relates to the pharmaceutical combination as defined above for its use for the treatment of cancer, by administration of the compound of formula (I) and the administration of the compound (2b).

According to an embodiment, the present invention relates to the pharmaceutical combination as defined above for its use for the treatment of cancer, by administration of the compound of formula (I) followed by the administration of the MAPK pathway inhibitor.

According to an embodiment, the present invention relates to the pharmaceutical combination as defined above for its use for the treatment of cancer, by administration of the MAPK pathway inhibitor followed by the administration of the compound of formula (I).

According to an embodiment, the present invention relates to the pharmaceutical combination as defined above for its use for the treatment of cancer, by administration of the compound of formula (I) followed by the administration of the compound (2a).

According to an embodiment, the present invention relates to the pharmaceutical combination as defined above for its use for the treatment of cancer, by administration of the compound of formula (I) followed by the administration of the compound (2b).

According to an embodiment, the present invention relates to the pharmaceutical combination as defined above for its use for the treatment of cancer, by administration of the compound (2a) followed by the administration of the compound of formula (I).

According to an embodiment, the present invention relates to the pharmaceutical combination as defined above for its use for the treatment of cancer, by administration of the compound (2b) followed by the administration of the

compound of formula (I).

According to an embodiment, the present invention relates to the pharmaceutical combination as defined above for its use for the treatment of cancer, wherein the compound of formula (I) and the MAPK pathway inhibitor are in amounts that produce a synergistic effect in reducing tumor volume.

According to an embodiment, the present invention relates to the pharmaceutical combination as defined above for its use for the treatment of cancer, wherein the compound of formula (I) and the MAPK pathway inhibitor are in amounts that produce a combined effect of tumor stasis.

According to an embodiment, the present invention relates to the combination as defined above, wherein the cancer is chosen from the group consisting of: non-small cell lung cancer, breast cancer, pancreatic cancer, liver cancer, prostate cancer, bladder cancer, cervical cancer, thyroid cancer, colorectal cancer, liver cancer, muscle cancer, hematological malignancies, melanoma, endometrial cancer and pancreatic cancer.

According to an embodiment, the present invention relates to the combination as defined above, wherein the cancer is chosen from the group consisting of: colorectal cancer, endometrial cancer, hematological malignancies, thyroid cancer, breast cancer, melanoma, pancreatic cancer and prostate cancer.

According to an embodiment, the present invention relates to the combination as defined above, wherein administration of the compound of formula (I) is followed by the administration of the MAPK pathway inhibitor.

According to an embodiment, the present invention relates to the combination as defined above, wherein administration of the MAPK pathway inhibitor is followed by the administration of the compound of formula (I).

According to an embodiment, the present invention relates to the combination as defined above, wherein administration of the compound of formula (I) is followed by the administration of the compound (2a).

According to an embodiment, the present invention relates to the combination as defined above, wherein administration of the compound of formula (I) is followed by the administration of the compound (2b).

According to an embodiment, the present invention relates to the combination as defined above, wherein administration of the compound (2a) is followed by the administration of the compound of formula (I).

According to an embodiment, the present invention relates to the combination as defined above, wherein administration of the compound (2b) is followed by the administration of the compound of formula (I).

According to an embodiment, the present invention relates to the combination as defined above, wherein the compound of formula (I) and the MAPK pathway inhibitor are in amounts that produce a synergistic effect in reducing tumor volume. According to an embodiment, the present invention relates to the combination as defined above, wherein the compound of formula (I) and the MAPK pathway inhibitor are in amounts that produce a combined effect of tumor stasis.

The present invention also relates to a pharmaceutical combination comprising:

- at least one compound of formula (I) as defined above, or a pharmaceutically acceptable salt thereof, and
- at least one MAPK pathway inhibitor chosen from the group consisting of the compound (2a) as defined above, or a pharmaceutically acceptable salt thereof, and the compound (2b) as defined above, or a pharmaceutically acceptable salt thereof,

  for its use for the treatment of cancer.

The present invention also relates to a product comprising:

- at least one compound of formula (I) as defined above or a pharmaceutically acceptable salt thereof, and
- at least one MAPK pathway inhibitor,

  as a combined preparation for simultaneous, separate or sequential use in anticancer therapy.

When the product as mentioned above is as a combined preparation for sequential use in anticancer therapy, either the compound of formula (I) is administered first and then the MAPK pathway inhibitor, or the MAPK is administered first and then the compound of formula (I).

In another aspect, methods of treating a patient with cancer are provided that comprise administering to the patient a therapeutically effective amount of a compound of Formula (I) as above indicated, or a pharmaceutically acceptable salt thereof, in combination with a compound selected from inhibitors of MAPK pathway, including the MEK and RAF inhibitors.

In another aspect, methods of treating a patient with cancer are provided that comprise administering to the patient a therapeutically effective amount of a compound of Formula (I) as above indicated, or a pharmaceutically acceptable salt thereof, in combination with a compound selected from inhibitors of MEK.

In another aspect, methods of treating a patient with cancer are provided that comprise administering to the patient a therapeutically effective amount of a compound of Formula (I) as above indicated, or a pharmaceutically acceptable salt thereof, in combination with a compound selected from inhibitors of RAF .

In one embodiment, a method of treating a patient with cancer comprises administering to the patient a dosage

of a MEK or RAF inhibitor and a dosage of a PI3Kβ inhibitor, wherein said PI3Kβ inhibitor has the above formula (I).

In one embodiment, a method of treating a patient with cancer comprises administering to the patient a dosage of a MEK inhibitor and a dosage of a PI3Kβ inhibitor, wherein said MEK inhibitor has the above-defined formula (2a), and the said PI3Kβ inhibitor has the above-defined formula (I).

In one embodiment, a method of treating a patient with cancer comprises administering to the patient a dosage of a RAF inhibitor and a dosage of a PI3Kβ inhibitor, wherein said RAF inhibitor has the formula (2b) as defined above, and the PI3Kβ inhibitor has the formula (I) as defined above.

In some embodiments, the compositions and methods of use described herein are in amounts (i.e., either in the composition are in an administered dosage) that synergistically reduce tumor volume in a patient. In further embodiments, the synergistic combination achieves tumor stasis or tumor regression.

In another aspect, kits are provided comprising: (A) a compound according to Formula (I) as defined above, or a pharmaceutically acceptable salt thereof; (B) a compound selected from the group consisting of Formula (2a) and Formula (2b) as defined above, or a pharmaceutically acceptable salt thereof; and optionally (C) instructions for use.

The present invention also relates to a kit comprising:

- at least one compound of formula (I) as defined above, or a pharmaceutically acceptable salt thereof,
- at least one MAPK pathway inhibitor, and
- optionally, instructions for use.

The present invention also relates to a kit comprising:

- at least one compound of formula (I) as defined above, or a pharmaceutically acceptable salt thereof,
- at least one MAPK pathway inhibitor chosen from the group consisting of the compound (2a) as defined above, or a pharmaceutically acceptable salt thereof, and the compound (2b) as defined above or a pharmaceutically acceptable salt thereof, and
- optionally, instructions for use.

Other objects, features and advantages will become apparent from the following detailed description. The detailed description and specific examples are given for illustration only since various changes and modifications within the spirit and scope of the invention will become apparent to those skilled in the art from this detailed description. Further, the examples demonstrate the principle of the invention and cannot be expected to specifically illustrate the application of this invention to all the examples where it will be obviously useful to those skilled in the prior art.

DETAILED DESCRIPTION

In one aspect, methods for treating patients with cancer are provided. In one embodiment, the methods comprise administering to the patient a therapeutically effective amount of a MAPK pathway inhibitors, including MEK and RAF inhibitors, and a therapeutically effective amount of a PI3Kβ inhibitor, as further described below.

In one aspect, methods for treating patients with cancer are provided. In one embodiment, the methods comprise administering to the patient a therapeutically effective amount of a MEK inhibitor and a therapeutically effective amount of a PI3Kβ inhibitor, as further described below.

In one aspect, methods for treating patients with cancer are provided. In one embodiment, the methods comprise administering to the patient a therapeutically effective amount of a RAF inhibitor and a therapeutically effective amount of a PI3Kβ inhibitor, as further described below.

In one embodiment, the MEK inhibitor has the structural formula (2a) as defined above.

The MEK inhibitor according to formula (2a) is referred to herein as "Compound of formula (2a)" and is known also as AZD6244. The preparation, properties, and MEK-inhibiting abilities of this compound are provided in, for example, International Patent Publication No. WO2003/077914, particularly Example 10 Compound 29c and Table p37 therein. The entire contents of WO2003/077914 are incorporated herein by reference. Neutral and salt forms of the compound of formula (2a) are all considered herein.

In one embodiment, the RAF inhibitor has the structural formula (2b) as defined above.

The RAF inhibitor according to formula (2b) is referred to herein as "compound of formula (2b)" and is known also as PLX4032. The preparation, properties, and RAF inhibiting abilities of compound (2b) are provided in, for example, International Patent Publication No. WO 2007/002325, particularly Example 44 compound P-0956 and Tables 2a, 2b, 2c, 2d, 2e and 2h therein. The entire contents of WO2007/002325 are incorporated herein by reference. Neutral and salt forms of the compound of Formula (2b) are all considered herein.

In one embodiment, the PI3Kβ inhibitor has the structural formula (I) as defined above.

The PI3Kβ inhibitor according to Formula (I) is referred to herein as "compound (I)" The preparation, properties,

and PI3Kβ-inhibiting abilities of compound (I) are provided in, for example, International Patent Publication No. WO2011/001114, particularly Example 117 and Table p 216 therein. The entire contents of W02011/001114 are incorporated herein by reference. Neutral and salt forms of the compound of Formula (I) are all considered herein.

In some embodiments, the compounds described above could be unsolvated. According to an embodiment, the compounds described above could be in solid forms. In other embodiments, one or both of the compounds used in the method are in solvated form. As known in the art, the solvate can be any of pharmaceutically acceptable solvent, such as water, ethanol, and the like. In general, the presence of a solvate or lack thereof does not have a substantial effect on the efficacy of the MEK or RAF or PI3Kβ inhibitor described above.

Although the compounds of formula (I), formula (2a) and formula (2b) are depicted in their neutral forms, in some embodiments, these compounds are used in a pharmaceutically acceptable salt form. The salt can be obtained by any of the methods well known in the art, such as any of the methods and salt forms elaborated upon in WO 2011/001114, as incorporated by reference herein.

A "pharmaceutically acceptable salt" of the compound refers to a salt that is pharmaceutically acceptable and that retains pharmacological activity. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, or S. M. Berge, et al., "Pharmaceutical Salts," J. Pharm. Sci., 1977; 66:1-19, both of which are incorporated herein by reference.

Examples of pharmaceutically acceptable acid addition salts include those formed with inorganic acids such as hydrochloric acid, hydrobromic acid, sulfuric acid, nitric acid, phosphoric acid, as well as those salts formed with organic acids, such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-I-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid.

In a first set of embodiments, the MEK inhibitor of formula (2a) is administered simultaneously with the PI3Kβ inhibitor of formula (I). Simultaneous administration typically means that both compounds enter the patient at precisely the same time. However, simultaneous administration also includes the possibility that the MEK inhibitor and PI3Kβ inhibitor enter the patient at different times, but the difference in time is sufficiently miniscule that the first administered compound is not provided the time to take effect on the patient before entry of the second administered compound. Such delayed times typically correspond to less than 1 minute, and more typically, less than 30 seconds.

In a first set of embodiments, the RAF inhibitor of formula (2b) is administered simultaneously with the PI3Kβ inhibitor of Formula (I). Simultaneous administration typically means that both compounds enter the patient at precisely the same time. However, simultaneous administration also includes the possibility that the RAF inhibitor and PI3Kβ inhibitor enter the patient at different times, but the difference in time is sufficiently miniscule that the first administered compound is not provided the time to take effect on the patient before entry of the second administered compound. Such delayed times typically correspond to less than 1 minute, and more typically, less than 30 seconds.

In one example, wherein the compounds are in solution, simultaneous administration can be achieved by administering a solution containing the combination of compounds. In another example, simultaneous administration of separate solutions, one of which contains the MEK inhibitor and the other of which contains the PI3Kβ inhibitor, can be employed. In one example wherein the compounds are in solid form, simultaneous administration can be achieved by administering a composition containing the combination of compounds.

In one example, wherein the compounds are in solution, simultaneous administration can be achieved by administering a solution containing the combination of compounds. In another example, simultaneous administration of separate solutions, one of which contains the RAF inhibitor and the other of which contains the PI3Kβ inhibitor, can be employed. In one example wherein the compounds are in solid form, simultaneous administration can be achieved by administering a composition containing the combination of compounds.

In one example, the compounds of the invention could be in solid form, in particular as tablets. In one embodiment, the compound (I) may be administered in solid form, in particular as a tablet.

In other embodiments, the MEK and PI3Kβ inhibitors are not simultaneously administered. In this regard, the first administered compound is provided time to take effect on the patient before the second administered compound is administered. Generally, the difference in time does not extend beyond the time for the first administered compound to complete its effect in the patient, or beyond the time the first administered compound is completely or substantially eliminated or deactivated in the patient. In one set of embodiments, the MEK inhibitor is administered before the PI3Kβ inhibitor. In another set of embodiments, the PI3Kβ inhibitor is administered before the MEK inhibitor. The time difference in non-simultaneous administrations is typically greater than 1 minute, and can be, for example, precisely, at least, up

to, or less than 5 minutes, 10 minutes, 15 minutes, 30 minutes, 45 minutes, 60 minutes, two hours, three hours, six hours, nine hours, 12 hours, 24 hours, 36 hours, or 48 hours, or more than 48 hours.

In other embodiments, the RAF and PI3Kβ inhibitors are not simultaneously administered. In this regard, the first administered compound is provided time to take effect on the patient before the second administered compound is administered. Generally, the difference in time does not extend beyond the time for the first administered compound to complete its effect in the patient, or beyond the time the first administered compound is completely or substantially eliminated or deactivated in the patient. In one set of embodiments, the RAF inhibitor is administered before the PI3Kβ inhibitor. In another set of embodiments, the PI3Kβ inhibitor is administered before the RAF inhibitor. The time difference in non-simultaneous administrations is typically greater than 1 minute, and can be, for example, precisely, at least, up to, or less than 5 minutes, 10 minutes, 15 minutes, 30 minutes, 45 minutes, 60 minutes, two hours, three hours, six hours, nine hours, 12 hours, 24 hours, 36 hours, or 48 hours, or more than 48 hours.

In one set of embodiments, one or both of the MEK and PI3Kβ inhibitors are administered in a therapeutically effective (i.e., therapeutic) amount or dosage. A "therapeutically effective amount" is an amount of the MEK or PI3Kβ inhibitor that, when administered to a patient by itself, effectively treats the cancer (for example, inhibits tumor growth, stops tumor growth, or causes tumor regression). An amount that proves "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the compound that corresponds to a therapeutically effective amount is strongly dependent on the type of cancer, stage of the cancer, the age of the patient being treated, and other facts. In general, therapeutically effective amounts of these compounds are well-known in the art, such as provided in the supporting references cited above.

In one set of embodiments, one or both of the RAF and PI3Kβ inhibitors are administered in a therapeutically effective (i.e., therapeutic) amount or dosage. A "therapeutically effective amount" is an amount of the RAF or PI3Kβ inhibitor that, when administered to a patient by itself, effectively treats the cancer (for example, inhibits tumor growth, stops tumor growth, or causes tumor regression). An amount that proves "therapeutically effective amount" in a given instance, for a particular subject, may not be effective for 100% of subjects similarly treated for the disease or condition under consideration, even though such dosage is deemed a "therapeutically effective amount" by skilled practitioners. The amount of the compound that corresponds to a therapeutically effective amount is strongly dependent on the type of cancer, stage of the cancer, the age of the patient being treated, and other facts. In general, therapeutically effective amounts of these compounds are well-known in the art, such as provided in the supporting references cited above.

In another set of embodiments, one or both of the MEK and PI3Kβ inhibitors are administered in a sub-therapeutically effective amount or dosage. A sub-therapeutically effective amount is an amount of the MEK or PI3Kβ inhibitor that, when administered to a patient by itself, does not completely inhibit over time the biological activity of the intended target.

In another set of embodiments, one or both of the RAF and PI3Kβ inhibitors are administered in a sub-therapeutically effective amount or dosage. A sub-therapeutically effective amount is an amount of the RAF or PI3Kβ inhibitor that, when administered to a patient by itself, does not completely inhibit over time the biological activity of the intended target.

Whether administered in therapeutic or sub-therapeutic amounts, the combination of MEK inhibitor and PI3Kβ inhibitor should be effective in treating the cancer. A sub-therapeutic amount of MEK inhibitor can be an effective amount if, when combined with the PI3Kβ inhibitor, the combination is effective in the treatment of a cancer.

Whether administered in therapeutic or sub-therapeutic amounts, the combination of RAF inhibitor and PI3Kβ inhibitor should be effective in treating the cancer. A sub-therapeutic amount of RAF inhibitor can be an effective amount if, when combined with the PI3Kβ inhibitor, the combination is effective in the treatment of a cancer.

In some embodiments, the combination of compounds exhibits a synergistic effect (i.e., greater than additive effect) in treating the cancer, particularly in reducing a tumor volume in the patient. In different embodiments, depending on the combination and the effective amounts used, the combination of compounds can either inhibit tumor growth, achieve tumor stasis, or even achieve substantial or complete tumor regression.

In some embodiments, as shown in the examples, Compound (I) can be administered at a dosage of about 100 mg/kg to 200 mg/kg po twice a day in tumor-bearing mice. Compound (2a), meanwhile, can be administered at a dosage of about 1 mg/kg to 50 mg/kg, preferably from 1 mg/kg to 30 mg/kg, po qd in tumor-bearing mice. Compound (2b) can be administered at a dosage of about 1 mg/kg to 150 mg/kg, preferably from 10 mg/kg to 100 mg/kg po qd in tumor-bearing mice.

In some embodiments, as shown in the examples, Compound (I) can be administered at a dosage of about 150 mg/kg po bi-daily in tumor-bearing mice. Compound (2a), meanwhile, can be administered at a dosage of about 10 mg/kg or 25 mg/kg po qd in tumor-bearing mice. Compound (2b) can be administered at a dosage of about 50 mg/kg or 100 mg/kg po qd in tumor-bearing mice.

According to an embodiment, as shown in the examples, the compound (I) can be administered twice a day.

According to an embodiment, as shown in the examples, the compounds (2a) and (2b) can be administered

once a day.

As used herein, the term "about" generally indicates a possible variation of no more than 10%, 5%, or 1% of a value. For example, "about 25 mg/kg" will generally indicate, in its broadest sense, a value of 22.5-27.5 mg/kg, i.e., 25 ± 2.5 mg/kg.

While the amounts of MEK, RAF and PI3Kβ inhibitors should result in the effective treatment of a cancer, the amounts, when combined, are preferably not excessively toxic to the patient (i.e., the amounts are preferably within toxicity limits as established by medical guidelines). In some embodiments, either to prevent excessive toxicity and/or provide a more efficacious treatment of the cancer, a limitation on the total administered dosage is provided. Typically, the amounts considered herein for example are per day; however, half-day and two-day or three-day cycles also are considered herein.

Different dosage regimens may be used to treat the cancer. In some embodiments, a daily dosage, such as any of the exemplary dosages described above, is administered once, twice, three times, or four times a day for at least three, four, five, six, seven, eight, nine, or ten days. Depending on the stage and severity of the cancer, a shorter treatment time (e.g., up to five days) may be employed along with a high dosage, or a longer treatment time (e.g., ten or more days, or weeks, or a month, or longer) may be employed along with a low dosage. In some embodiments, a once- or twice-daily dosage is administered every other day. In some embodiments, each dosage contains both the MEK and PI3Kβ inhibitors, while in other embodiments, each dosage contains either the MEK or PI3Kβ inhibitors. In yet other embodiments, some of the dosages contain both the MEK and PI3Kβ inhibitors, while other dosages contain only the MEK or the PI3Kβ inhibitor.

In some embodiments, each dosage contains both the RAF and PI3Kβ inhibitors, while in other embodiments, each dosage contains either the RAF or PI3Kβ inhibitors. In yet other embodiments, some of the dosages contain both the BRAF and PI3Kβ inhibitors, while other dosages contain only the RAF or the PI3Kβ inhibitor.

Examples of types of cancers to be treated with the present invention include, but are not limited to, lymphomas, sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, synovioma, mesothelioma, lymphangioendotheliosarcoma, Ewing's tumor, leiomyosarcoma, rhabdomyosarcoma, colon carcinoma, pancreatic cancer, breast cancer, ovarian cancer, prostate cancer, gastric cancer, esophageal cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, non-small cell lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; thyroid cancer, endometrial cancers; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia and heavy chain disease.

In some embodiments, the cancer being treated is selected from the group consisting of non-small cell lung cancer, breast cancer, pancreatic cancer, liver cancer, prostate cancer, bladder cancer, cervical cancer, thyroid cancer, colorectal cancer, liver cancer, and muscle cancer. In other embodiments, the cancer is selected from colorectal cancer, endometrial cancer, hematology cancer, thyroid cancer, triple negative breast cancer, prostate or melanoma.

The patient considered herein is typically a human. However, the patient can be any mammal for which cancer treatment is desired. Thus, the methods described herein can be applied to both human and veterinary applications.

The term "treating" or "treatment", as used herein, indicates that the method has, at the least, mitigated abnormal cellular proliferation. For example, the method can reduce the rate of tumor growth in a patient, or prevent the continued growth of a tumor, or even reduce the size of a tumor.

In another aspect, methods for preventing cancer in an animal are provided. In this regard, prevention denotes causing the clinical symptoms of the disease not to develop in an animal that may be exposed to or predisposed to the disease but does not yet experience or display symptoms of the disease. The methods comprise administering to the patient a MEK inhibitor and a PI3Kβ inhibitor, as described herein. The methods comprise administering to the patient in need thereof a RAF inhibitor and a PI3Kβ inhibitor, as described herein. In one example, a method of preventing cancer in an animal comprises administering to the animal a compound of formula (I), or a pharmaceutically acceptable salt thereof, in combination with a compound selected from the group consisting of formula (2a) and formula (2b), or a pharmaceutically acceptable salt thereof.

The MEK and PI3Kβ inhibiting compounds, or their pharmaceutically acceptable salts or solvate forms, in pure form or in an appropriate pharmaceutical composition, can be administered via any of the accepted modes of administration or agents known in the art. The compounds can be administered, for example, orally, nasally, parenterally (intravenous, intramuscular, or subcutaneous), topically, transdermally, intravaginally, intravesically, intracistemally, or

rectally. The dosage form can be, for example, a solid, semi-solid, lyophilized powder, or liquid dosage forms, such as for example, tablets, pills, soft elastic or hard gelatin capsules, powders, solutions, suspensions, suppositories, aerosols, or the like, preferably in unit dosage forms suitable for simple administration of precise dosages. A particular route of administration is oral, particularly one in which a convenient daily dosage regimen can be adjusted according to the degree of severity of the disease to be treated.

In another aspect, the instant application is directed to a composition that includes the MEK inhibitor of formula (2a) and a PI3Kβ inhibitor of formula (I). In another aspect, the instant application is directed to a composition that includes the RAF inhibitor of formula (2b) and a PI3Kβ inhibitor of formula (I). In some embodiments, the composition includes only the MEK and PI3Kβ inhibitors described above. In some embodiments, the composition includes only the RAF and PI3Kβ inhibitors described above. In other embodiments, the composition is in the form of a solid (e.g., a powder or tablet) including the MEK and PI3Kβ inhibitors in solid form, and optionally, one or more auxiliary (e.g., adjuvant) or pharmaceutically active compounds in solid form. In other embodiments, the composition further includes any one or combination of pharmaceutically acceptable carriers (i.e., vehicles or excipients) known in the art, thereby providing a liquid dosage form. In other embodiments, the composition is in the form of a solid (e.g., a powder or tablet) including the RAF and PI3Kβ inhibitors in solid form, and optionally, one or more auxiliary (e.g., adjuvant) or pharmaceutically active compounds in solid form. In other embodiments, the composition further includes any one or combination of pharmaceutically acceptable carriers (i.e., vehicles or excipients) known in the art, thereby providing a liquid dosage form.

Auxiliary and adjuvant agents may include, for example, preserving, wetting, suspending, sweetening, flavoring, perfuming, emulsifying, and dispensing agents. Prevention of the action of microorganisms is generally provided by various antibacterial and antifungal agents, such as, parabens, chlorobutanol, phenol, sorbic acid, and the like. Isotonic agents, such as sugars, sodium chloride, and the like, may also be included. Prolonged absorption of an injectable pharmaceutical form can be brought about by the use of agents delaying absorption, for example, aluminum monostearate and gelatin. The auxiliary agents also can include wetting agents, emulsifying agents, pH buffering agents, and antioxidants, such as, for example, citric acid, sorbitan monolaurate, triethanolamine oleate, butylated hydroxytoluene, and the like.

Dosage forms suitable for parenteral injection may comprise physiologically acceptable sterile aqueous or non-aqueous solutions, dispersions, suspensions or emulsions, and sterile powders for reconstitution into sterile injectable solutions or dispersions. Examples of suitable aqueous and non-aqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch, alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms also may comprise buffering agents.

Solid dosage forms as described above can be prepared with coatings and shells, such as enteric coatings and others well-known in the art. They can contain pacifying agents and can be of such composition that they release the active compound or compounds in a certain part of the intestinal tract in a delayed manner. Examples of embedded compositions that can be used are polymeric substances and waxes. The active compounds also can be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., a MEK , RAF or PI3Kβ inhibitor compound described herein, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethyl formamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, may contain suspending agents, as for example, ethoxylated

isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are, for example, suppositories that can be prepared by mixing the compounds described herein with, for example, suitable non-irritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration may include, for example, ointments, powders, sprays, and inhalants. The active component is admixed under sterile conditions with a physiologically acceptable carrier and any preservatives, buffers, or propellants as can be required. Ophthalmic formulations, eye ointments, powders, and solutions also can be employed.

Generally, depending on the intended mode of administration, the pharmaceutically acceptable compositions will contain about 1% to about 99% by weight of the compounds described herein, or a pharmaceutically acceptable salt thereof, and 99% to 1% by weight of a pharmaceutically acceptable excipient. In one example, the composition will be between about 5% and about 75% by weight of a compounds described herein, or a pharmaceutically acceptable salt thereof, with the rest being suitable pharmaceutical excipients.

Actual methods of preparing such dosage forms are known, or will be apparent, to those skilled in this art. Reference is made, for example, to

Remington's Pharmaceutical Sciences, 18th Ed., (Mack Publishing Company, Easton, Pa., 1990).

In some embodiments, the composition does not include one or more other anti-cancer compounds. In other embodiments, the composition includes one or more other anti-cancer compounds. For example, administered compositions can comprise standard of care agents for the type of tumors selected for treatment.

In another aspect, kits are provided. Kits according to the invention include package(s) comprising compounds or compositions of the invention. In one embodiment, kits comprise compound (I), or a pharmaceutically acceptable salt thereof, and a compound selected from the group consisting of compound (2a) and compound (2b), or a pharmaceutically acceptable salt thereof.

The phrase "package" means any vessel containing compounds or compositions presented herein. In some embodiments, the package can be a box or wrapping. Packaging materials for use in packaging pharmaceutical products are well-known to those of skill in the art. Examples of pharmaceutical packaging materials include, but are not limited to, bottles, tubes, inhalers, pumps, bags, vials, containers, syringes, bottles, and any packaging material suitable for a selected formulation and intended mode of administration and treatment.

The kit also can contain items that are not contained within the package but are attached to the outside of the package, for example, pipettes.

Kits can contain instructions for administering compounds or compositions of the invention to a patient. Kits also can comprise instructions for approved uses of compounds herein by regulatory agencies, such as the United States Food and Drug Administration. Kits also can contain labeling or product inserts for the inventive compounds. The package(s) and/or any product insert(s) may themselves be approved by regulatory agencies. The kits can include compounds in the solid phase or in a liquid phase (such as buffers provided) in a package. The kits also can include buffers for preparing solutions for conducting the methods, and pipettes for transferring liquids from one container to another.

Examples have been set forth below for the purpose of illustration and to describe certain specific embodiments of the invention. However, the scope of the claims is not to be in any way limited by the examples set forth herein.

## BRIEF DESCRIPTION OF THE DRAWINGS

**Figure 1** is an isobologram representation of the *in vitro* activity of compound (I) in combination with compound (2a) in human melanoma cell line UACC-62.

**Figure 2** is an isobologram representation of the *in vitro* activity of compound (I) in combination with compound (2b) in human melanoma cell line UACC-62.

**Figure 3** is an isobologram representation of the *in vitro* activity of compound (I) in combination with compound (2a) in human melanoma cell line WM-266.4.

**Figure 4** is an isobologram representation of the *in vitro* activity of compound (I) in combination with compound (2b) in human melanoma cell line WM-266.4.

**Figure 5** provides a plot showing body weight change during the evaluation of the antitumor activity of compound

(I) (150 mg/kg bid) in combination with compound (2a)(AZD-6244)(10 and 25 mg/kg qd) against human melanoma tumors UACC-62 bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

**Figure 6** provides a plot showing antitumor activity of compound (I) (150 mg/kg bid) in combination with compound (2a) (AZD-6244)(10 and 25 mg/kg qd) against human melanoma tumors UACC-62 bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

**Figure 7** provides a plot showing body weight change during the evaluation of the antitumor activity of compound (I) (151.5 mg/kg bid) in combination with compound (2b) (PLX-4032)(50 and 100 mg/kg qd) against human melanoma tumors UACC-62 bearing SCID female mice.
The curve with white squares corresponds to control; the curve with continuous line corresponds to compound (I) at 151.5 mg/kg twice a day; the curve with dotted line and black triangles corresponds to compound (2b) at 100 mg/kg once a day; the curve with dotted line and black lozenges corresponds to compound (2b) at 50 mg/kg once a day; the curve with continuous line and black triangles corresponds to the combination of compound (I) at 151.5 mg/kg twice a day and compound (2b) at 100 mg/kg once a day; the curve with continuous line and black lozenges corresponds to the combination of compound (I) at 151.5 mg/kg twice a day and compound (2b) at 50 mg/kg once a day; and the black triangles curve corresponds to the treatment PO.

**Figure 8** provides a plot showing antitumor activity of compound (I) (151.5 mg/kg bid) in combination with compound (2b) (PLX-4032)(50 and 100 mg/kg qd) against human melanoma tumors UACC-62 bearing SCID female mice.
The curve with white squares corresponds to control; the curve with continuous line corresponds to compound (I) at 151.5 mg/kg twice a day; the curve with dotted line and black triangles corresponds to compound (2b) at 100 mg/kg once a day; the curve with dotted line and black lozenges corresponds to compound (2b) at 50 mg/kg once a day; the curve with continuous line and black triangles corresponds to the combination of compound (I) at 151.5 mg/kg twice a day and compound (2b) at 100 mg/kg once a day; the curve with continuous line and black lozenges corresponds to the combination of compound (I) at 151.5 mg/kg twice a day and compound (2b) at 50 mg/kg once a day; and the black triangles curve corresponds to the treatment PO.

**Figure 9** provides a plot showing body weight change during the evaluation of the antitumor activity of compound (I) (150 mg/kg bid) in combination with compound (2a) (AZD-6244)(10 and 25 mg/kg qd) against human melanoma tumors WM-266.4 bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

**Figure 10** provides a plot showing antitumor activity of compound (I) (150 mg/kg bid) in combination with compound (2a) (AZD-6244)(10 and 25 mg/kg qd) against human melanoma tumors WM-266.4 bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg

twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

**Figure 11** provides a plot showing body weight change during the evaluation of the antitumor activity of compound (I) (150 mg/kg bid) in combination with compound (2b) (PLX-4032)(50 and 100 mg/kg qd) against human melanoma tumors WM-266.4 bearing SCID female mice.
The curve with white squares corresponds to control; the curve with continuous line corresponds to compound (I) at 150 mg/kg twice a day; the curve with dotted line and black triangles corresponds to compound (2b) at 100 mg/kg once a day; the curve with dotted line and black lozenges corresponds to compound (2b) at 50 mg/kg once a day; the curve with continuous line and black triangles corresponds to the combination of compound (I) at 150 mg/kg twice a day and compound (2b) at 100 mg/kg once a day; the curve with continuous line and black lozenges corresponds to the combination of compound (I) at 150 mg/kg twice a day and compound (2b) at 50 mg/kg once a day; and the black triangles curve corresponds to the treatment PO.

**Figure 12** provides a plot showing antitumor activity of compound (I) (150 mg/kg bid) in combination with compound (2b) (PLX-4032)(50 and 100 mg/kg qd) against human melanoma tumors WM-266.4 bearing SCID female mice.
The curve with white squares corresponds to control; the curve with continuous line corresponds to compound (I) at 150 mg/kg twice a day; the curve with dotted line and black triangles corresponds to compound (2b) at 100 mg/kg once a day; the curve with dotted line and black lozenges corresponds to compound (2b) at 50 mg/kg once a day; the curve with continuous line and black triangles corresponds to the combination of compound (I) at 150 mg/kg twice a day and compound (2b) at 100 mg/kg once a day; the curve with continuous line and black lozenges corresponds to the combination of compound (I) at 150 mg/kg twice a day and compound (2b) at 50 mg/kg once a day; and the black triangles curve corresponds to the treatment PO.

**Figure 13** provides a plot showing body weight change during the evaluation of the antitumor activity of compound (I) (150 mg/kg bid) in combination with compound (2a) (AZD-6244)(10 and 25 mg/kg qd) against human primary colon tumors CR-IGR-014P bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

**Figure 14** provides a plot showing antitumor activity of compound (I) (150 mg/kg bid) in combination with compound (2a) (AZD-6244)(10 and 25 mg/kg qd) against human primary colon tumors CR-IGR-014P bearing SCID female mice.
The curve with white squares corresponds to control; the curve with continuous line corresponds to compound (I) at 150 mg/kg twice a day; the curve with dotted line and black triangles corresponds to compound (2a) at 25 mg/kg once a day; the curve with dotted line and black lozenges corresponds to compound (2a) at 10 mg/kg once a day; the curve with continuous line and black triangles corresponds to the combination of compound (I) at 150 mg/kg twice a day and compound (2a) at 25 mg/kg once a day; the curve with continuous line and black lozenges corresponds to the combination of compound (I) at 150 mg/kg twice a day and compound (2a) at 10 mg/kg once a day; and the black triangles curve corresponds to the treatment PO.

## EXAMPLES

Several *in vitro* experiments have been conducted in order to study the interaction between a PI3Kβ inhibitor (compound I) with MEK inhibitors (here compound 2a) or with RAF inhibitors (here compound 2b) on the inhibitory activity on cell proliferation in human melanoma cell lines UACC-62 and WM-266.4 (BRAF mutant and PTEN deficient).

The interaction between compound (I) and compound (2a) or compound (2b) on both cell lines was characterized using ray design approach as described in R.Straetemans, (Biometrical Journal, 47, 2005) which allows to investigate synergy for different effective fraction fi of the compounds in the mixture, the effective fraction being constant for each ray. Representative experiments for each combination and each cell line are presented hereunder.

**Example 1:** *In vitro* activity of compound (I) in combination with compound (2a) (AZD-6244) in human melanoma cell lines UACC-62

To evaluate the anti-proliferative activity of the PI3Kβ selective inhibitor of formula (I), in combination with the MEK inhibitor AZD-6244 of formula (2a), experiments were conducted using human melanoma cell lines UACC-62 (BRAF mutant and PTEN-deficient). Prior to *in vitro* combination studies, the activity of individual agents was investigated using UACC-62 cell line. The purpose of testing individual agents was to determine the independence of their action and to determine the dilution design of the Fixed Ratio Drug Combination assay.

*Materials and methods*

The human melanoma UACC-62 cell line was purchased at NCI (Batch 0503000). The UACC-62 cells were cultured in RPMI1640 medium supplemented with 10% FBS and 2mM L-Glutamine.

Compound (I) and compound (2a) were dissolved in DMSO at concentration of 30 mM. They were diluted in cascade, in DMSO and then diluted 50-fold in culture medium containing 10% serum before being added onto cells with a 20-fold dilution factor. The final concentrations tested were defined by Ray design described in Table 1. The DMSO concentration was 0.1 % in controls and in all treated wells.

Table 1 provides the ray design used to perform the example 1 study.

Ray 1: Compound (I) alone

| | | | | | | | | | | |
|---|---|---|---|---|---|---|---|---|---|---|
| (I) | 30000 | 10000 | 3000 | 1000 | 300 | 100 | 30 | 10 | 3 | 1 |
| (2a) | 0 | 0 | 0 | 0 | 0 | 0 | 0 | 0 | 0 | 0 |

Ray 2 (≈1 for 3): f=0.09

| | | | | | | | | | | |
|---|---|---|---|---|---|---|---|---|---|---|
| (I) | 30000 | 10000 | 3000 | 1000 | 300 | 100 | 30 | 10 | 3 | 1 |
| (2a) | 1000 | 300 | 100 | 30 | 10 | 3 | 1 | 0.3 | 0.1 | 0.03 |

Ray 3 (≈1 for 1): f=0.23

| | | | | | | | | | | |
|---|---|---|---|---|---|---|---|---|---|---|
| (I) | 30000 | 10000 | 3000 | 1000 | 300 | 100 | 30 | 10 | 3 | 1 |
| (2a) | 300 | 100 | 30 | 10 | 3 | 1 | 0.3 | 0.1 | 0.03 | 0.01 |

Ray 4 (≈ 3 for 1): f=0.49

| | | | | | | | | | | |
|---|---|---|---|---|---|---|---|---|---|---|
| (I) | 30000 | 10000 | 3000 | 1000 | 300 | 100 | 30 | 10 | 3 | 1 |
| (2a) | 100 | 30 | 10 | 3 | 1 | 0.3 | 0.1 | 0.03 | 0.01 | 0.003 |

Ray 5: Compound (I) alone

| | | | | | | | | | | |
|---|---|---|---|---|---|---|---|---|---|---|
| (I) | 0 | 0 | 0 | 0 | 0 | 0 | 0 | 0 | 0 | 0 |
| (2a) | 10000 | 3000 | 1000 | 300 | 100 | 30 | 10 | 3 | 1 | 0.3 |

UACC-62 cells were plated at 2500 cells/well in 96-well plates in appropriate culture medium and incubated 6 hours at 37°C, 5% $CO_2$. Cells were treated in a grid manner with increasing concentrations of compound (I) ranging from 1 to 30,000 nM and with increasing concentrations of compound (2a) ranging from 0.001 to 10,000 nM, depending

on the given drug ratio, and incubated for 96 hours. Cell growth was evaluated by measuring intracellular ATP using CelltiterGlo® reagent (Promega) according to the manufacturer's protocol. Briefly, Cell Titer Glo was added to each plate, incubated for 1 hour then luminescent signal was read on the MicroBeta Luminescent plate reader. All plates were run in duplicate. All assays were run at least in duplicate.

Inhibition of cell growth was estimated after treatment with compound or combination of compounds for four days and comparing the signal to cells treated with vehicle (DMSO).

Growth inhibition percentage (GI%) was calculated according to the following equation:

$$GI\% = 100 * (1- ((X-BG) / (TC-BG))$$

where the values are defined as:

X = Value of wells containing cells in the presence of compounds A and B alone or in combination
BG = Value of wells with medium and without cells
TC = value of wells containing cells in the presence of vehicle (DMSO).

These measurements allow determining the potential synergistic combinations in using the following statistical method:

A global non linear mixed model using NLMIXED procedure of SAS V9.2 was applied to fit simultaneously the concentration-responses curves for each ray. The combination index

$$E(Y) = E\min + \frac{E\max - E\min}{1 + \exp(-m\log(\frac{Conc}{IC50}))}$$

of each ray i and its 95% confidence interval was then estimated using the following equation:

$$\frac{C_A}{IC40_A} + \frac{C_B}{IC40_B} = K_i$$

where $IC40_A$ and $IC40_B$ are the concentrations of compound A and compound B necessary to obtain 40% of inhibition for each compound alone and $C_A$ and $C_B$ are the concentrations of compound A and compound B in the mixture necessary to obtain 40% of inhibition.

Additivity was then concluded when the confidence interval of the interaction index (Ki) includes 1, synergy was concluded when the upper confidence interval bound is less than 1 and antagonism was concluded when the lower confidence interval bound is higher than 1.

The isobologram representation (Figure 1) permits to visualize the position of each ray according to the additivity situation represented by the straight-line joining ray 1 to ray 5. All rays below this line correspond to a potential synergistic situation whereas all rays above the line correspond to a potential antagonistic situation.

Results of *in vitro* studies

Compound (I), as single agent, inhibited the proliferation of UACC-62 cells with an IC40 of 3,630 nM. compound (2a), as single agent, inhibited the proliferation of UACC-62 cells with an IC40 of 27 nM (see table 2 below).

***Table 2: Absolute IC40 estimations for each compound alone in example 1***

| Absolute IC40 of single agents are estimated with 4-parameter logistic models | |
|---|---|
| | Absolute IC40s (nM) |
| Compound (I) | 3630.4 [2328.6 ; 4932.2] |

(continued)

| Absolute IC40 of single agents are estimated with 4-parameter logistic models | |
|---|---|
| | Absolute IC40s (nM) |
| Compound (2a) | 27.4 [22.0 ; 32.8] |

As described by the isobologram representation in Figure 1, in the combination arms, synergy was observed at near equipotent concentrations (ratio 1 /1 (f=0.43); Ray 3 & ratio 2/1 (f=0.71); Ray 4) and at concentrations so that compound (2a) was 4 times more effective than compound (I) (ratio ¼ (f=0.19); Ray 2), with Ki of 0.34 [0.24-0.44], 0.54 [0.36-0.73] and 0.35 [0.26-0.44], respectively (see below table 3)

These data correspond to a representative study out of 3 independent experiments. For these three experiments, synergy or additivity with tendency to synergy was observed for an effective fraction f between 0.10 and 0.80.

***Table 3: Interaction characterization in example 1***

| $K_i$ indexes allow the definition of the interaction observed between the two compounds. | | | |
|---|---|---|---|
| | **f values** | **Ki (confidence interval at 95%)** | **Interaction characterization** |
| **Ray 2** | 0.19 | 0.3504 [0.2561; 0.4446] | Synergy |
| **Ray 3** | 0.43 | 0.3413 [0.2379; 0.4448] | Synergy |
| **Ray 4** | 0.71 | 0.5443 [0.3627 ; 0.7260] | Synergy |

In the studied domain, synergy is observed when f is equal to 0.19, 0.43 and 0.71.

**Example 2:** *In vitro* activity of compound (I) in combination with compound (2b) in human melanoma cell lines UACC-62

To evaluate the anti proliferative activity of the PI3Kβ selective inhibitor of formula (I) in combination with the RAF inhibitor of formula (2b), experiments were conducted using human melanoma cell lines UACC-62 (BRAF mutant and PTEN-deficient). Prior to *in vitro* combination studies, the activity of individual agents was investigated using UACC-62 cell line. The purpose of testing individual agents was to determine the independence of their action and to determine the dilution design of the Fixed Ratio Drug Combination assay.

Materials and methods

Compound (I) and compound (2b) solutions were prepared according to the material and methods of example 1 and following the Ray design described in Table 4 below.

***Table 4: Ray Design of example 2***

Ray 1: Compound (I) alone

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 0 | 0 | 0 | 0 | 0 | 0 | 0 | 0 | 0 | 0 |

Ray 2 (≈1 for 3): f=0.31

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 300 | 100 | 30 | 10 | 3 | 1 | 0.3 | 0.1 | 0.03 | 0.01 |

Ray 3 (≈ 1 for 1): f=0.59

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 100 | 30 | 10 | 3 | 1 | 0.3 | 0.1 | 0.03 | 0.01 | 0.003 |

Ray 4 (≈3 for 1): f=0.82

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 30 | 10 | 3 | 1 | 0.3 | 0.1 | 0.03 | 0.01 | 0.003 | 0.001 |

Ray 5: Compound 2b alone

| | | | | | | | | | | |
|---|---|---|---|---|---|---|---|---|---|---|
| P1 | 0 | 0 | 0 | 0 | 0 | 0 | 0 | 0 | 0 | 0 |
| P2 | 10 | 3 | 1 | 0.3 | 0.1 | 30 | 10 | 3 | 1 | 0.3 |

Materials and methods are the same as described in example 1.

Results of *in vitro* studies

Compound (I), as single agent, inhibited the proliferation of UACC-62 cells with an IC40 of 17,700 nM. Compound (2b), as single agent, inhibited the proliferation of UACC-62 cells with an IC40 of 18 nM (Table 5).

***Table 5: Absolute $IC_{40}$ estimations for each compound alone in example 2***

| Absolute $IC_{40}$ of single agents are estimated with 4-parameter logistic models | |
|---|---|
| | Absolute $IC_{40}$ (nM) |
| Compound (I) | 17705 |
| Compound (2b) | 17.7 |

As described by the isobologram of Figure 2, in the combination rays, synergy was observed at equipotent concentrations (Ray 4 (f=0.50)) and at concentration so that compound (2b) was more effective than compound (I)(ratio 1/10 (f=0.09), Ray 2 and ratio 1/3 (f=0.24); Ray 3), with Ki of 0.56 [0.30-0.81], 0.57 [0.40-0.74] and 0.38 [0.25-0.52] respectively (Table 6).

***Table 6**: **interaction characterization in example 2***

| $K_i$ indexes allow us to define the interaction observed between the two compounds | | | |
|---|---|---|---|
| | **f values** | **Ki (confidence interval at 95%)** | **Interaction characterization** |
| **Ray 2** | 0.09 | 0.5733 [0.4019 ; 0.7448] | Synergy |

(continued)

| $K_i$ indexes allow us to define the interaction observed between the two compounds | | | |
|---|---|---|---|
| | **f values** | **Ki (confidence interval at 95%)** | **Interaction characterization** |
| **Ray 3** | 0.24 | 0.3845 [0.2494 ; 0.5196] | Synergy |
| **Ray 4** | 0.50 | 0.5554 [0.3048 ; 0.8059] | Synergy |

In the studied domain, synergy is observed when f is equal to 0.09, 0.24 and 0.50.

These data correspond to a representative study out of 3 independent experiments. For these three experiments, synergy or additivity with tendency to synergy was obtained for all proportions of compound (I) and compound (2b) in the mixture.

**Example 3**: *In vitro* activity of compound (I) in combination with compound (2a) in human melanoma cell line WM-266-4

To evaluate the anti proliferative activity of the PI3Kβ selective inhibitor compound (I) in combination with the MEK inhibitor compound (2a), experiments were conducted using human melanoma cell lines WM-266-4 (BRAF mutant and PTEN-deficient). Prior to *in vitro* combination studies, the activity of individual agents was investigated using WM-266-4 cell line. The purpose of testing individual agents was to determine the independence of their action and to determine the dilution design of the Fixed Ratio Drug Combination assay. The characterization of the interaction between compound (I) and compound (2a) was studied using the ray design method and associated statistical analysis, which evaluates the benefit of the combination at different drug efficacy ratios.

Materials and methods

The human melanoma WM-266-4 cell line was purchased at ATCC (Ref number CRL-1676 Batch 3272826). The WM-266-4 cells were cultured in RPMI1640 medium supplemented with 10% FBS and 2mM L-Glutamine.

Compounds (I) and (2a) dilutions were prepared according to the material and methods of example 1 and following the Ray design described in Table 7 below.

Materials and methods are the same described in example 1.

***Table 7: Ray Design proposal of Example 3***

Ray 1: Compound I alone

| | | | | | | | | | | |
|---|---|---|---|---|---|---|---|---|---|---|
| Compound (I) | 30000 | 10000 | 3000 | 1000 | 300 | 100 | 30 | 10 | 3 | 1 |
| Compound (2a) | 0 | 0 | 0 | 0 | 0 | 0 | 0 | 0 | 0 | 0 |

Ray 2 (≈1 for 3): f=0.16

| | | | | | | | | | | |
|---|---|---|---|---|---|---|---|---|---|---|
| Compound (I) | 30000 | 10000 | 3000 | 1000 | 300 | 100 | 30 | 10 | 3 | 1 |
| Compound (2a) | 3000 | 1000 | 300 | 100 | 30 | 10 | 3 | 1 | 0.3 | 0.1 |

Ray 3 (≈1 for 1): f=0.38

| | | | | | | | | | | |
|---|---|---|---|---|---|---|---|---|---|---|
| Compound (I) | 30000 | 10000 | 3000 | 1000 | 300 | 100 | 30 | 10 | 3 | 1 |
| Compound (2a) | 300 | 300 | 30 | 30 | 3 | 3 | 1 | 0.3 | 0.1 | 0.03 |

Ray 4 (≈ 3 for 1), f=0.66

| | | | | | | | | | | |
|---|---|---|---|---|---|---|---|---|---|---|
| Compound (I) | 30000 | 10000 | 3000 | 1000 | 300 | 100 | 30 | 10 | 3 | 1 |
| Compound (2a) | 300 | 100 | 30 | 10 | 3 | 1 | 0.3 | 0.1 | 0.03 | 0.01 |

Ray 5: compound I alone

| | | | | | | | | | | |
|---|---|---|---|---|---|---|---|---|---|---|
| Compound (I) | 0 | 0 | 0 | 0 | 0 | 0 | 0 | 0 | 0 | 0 |
| Compound (2a) | 10000 | 3000 | 1000 | 300 | 100 | 30 | 10 | 3 | 1 | 0.3 |

Results of *in vitro* studies

Compound (I), as single agent, inhibited the proliferation of WM-266-4 cells with an IC40 of 834 nM. Compound (2a), as single agent, inhibited the proliferation of WM-266-4 cells with an IC40 of 33 nM (Table 8).

***Table 8: Absolute $IC_{40}$ estimations for each compound alone in example 3***

| Absolute $IC_{40}$ of single agents are estimated with 4-parameter logistic models | |
|---|---|
| | Absolute $IC_{40}$ (nM) |
| Compound (I) | 834.0 [409; 1259.0] |
| Compound (2a) | 33.5 [29.5; 37.4] |

As described by the isobologram representation in Figure 3, in the combination arms, synergy was observed at equipotent concentrations (ratio 1/1 (f=0.56): Ray 3) and at concentrations so that compound (2a) was 3 times more effective than compound (I)(ratio 1/3 (f=0.29): Ray 2), with Ki of 0.50 [0.34-0.66] and 0.43 [0.33-0.53], respectively.

Additivity was observed in the domain so that compound (I) was 4 times more effective than compound (2a)(Ray 4 (f=0.80)), with a Ki of 1.01 [0.57-1.44] (Table 9).

***Table 9: Interaction characterization in example 3***

| Interaction indexes (Ki) allow us to define the interaction observed between the two compounds. | | | |
|---|---|---|---|
| | **f values** | **Ki (confidence interval at 95%)** | **Interaction characterization** |
| **Ray 2** | 0.29 | 0.4340 [0.3341 ; 0.5339] | Synergy |
| **Ray 3** | 0.56 | 0.5002 [0.3364 ; 0.6640] | Synergy |
| **Ray 4** | 0.80 | 1.0078 [0.5734 ; 1.4422] | Additivity |

In the studied domain, synergy is observed when f is equal to 0.29 and 0.56.

These data correspond to a representative study out of 3 independent experiments.

For these three experiments, synergy or additivity with tendency to synergy was observed for an effective fraction f between 0.28 and 0.56.

**Example 4**: <u>*In vitro* activity of compound (I) in combination with compound</u>

<u>(2b) in human melanoma cell line WM-266.4</u>

To evaluate the anti proliferative activity of the PI3Kβ selective inhibitor compound (I) in combination with the RAF inhibitor compound (2b), experiments were conducted using human melanoma cell lines WM-266.4 (BRAF mutant and PTEN-deficient). Prior to *in vitro* combination studies, the activity of individual agents was investigated using WM-266.4 cell line. The purpose of testing individual agents was to determine the independence of their action and to determine the dilution design of the Fixed Ratio Drug Combination assay. The characterization of the interaction between compound (I) and compound (2b) was studied using the ray design method and associated statistical analysis, which evaluates the benefit of the combination at different drug efficacy ratios.

<u>Materials and methods</u>

The human melanoma WM-266-4 cell line was purchased at ATCC (Ref number CRL-1676 Batch 3272826). The WM-266.4 cells were cultured in RPMI1640 medium supplemented with 10% FBS and 2mM L-Glutamine.

Compounds (I) and (2b) dilutions were prepared according to the material and methods of example 1 and following the Ray design described in Table 10 below.

Materials and methods are the same described in example 1.

***Table 10: Ray Design of Example 4***

Ray 1 : Compound (I) alone

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 0 | 0 | 0 | 0 | 0 | 0 | 0 | 0 | 0 | 0 |

Ray 2 (≈1 for 3): f=0.29

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 300 | 100 | 30 | 10 | 3 | 1 | 3 | 1 | 0.0 | 0.1 |

Ray 3 (≈1 for 1): f=0.56

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 300 | 30 | 30 | 3 | 3 | 1 | 0.3 | 0.1 | 0.03 | 0.01 |

Ray 4 (≈3 for 1), f=0.80

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 300 | 100 | 30 | 10 | 3 | 1 | 0.3 | 0.1 | 0.03 | 0.01 |

Ray 4 bis (≈9 for 1), f=0.93

| | | | | | | | | | | |
|---|---|---|---|---|---|---|---|---|---|---|
| P1 | 30000 | 10000 | 3000 | 1000 | 300 | 100 | 30 | 10 | 3 | 1 |
| P2 | 100 | 30 | 10 | 3 | 1 | 0.3 | 0.1 | 0.03 | 0.01 | 0.003 |

Ray 5 : Compound 2b alone

| | | | | | | | | | | |
|---|---|---|---|---|---|---|---|---|---|---|
| P1 | 0 | 0 | 0 | 0 | 0 | 0 | 0 | 0 | 0 | 0 |
| P2 | 10000 | 3000 | 1000 | 300 | 100 | 30 | 10 | 3 | 1 | 0.3 |

Results of *in vitro* studies

Compound (I), as single agent, inhibited the proliferation of UACC-62 cells with an IC40 of 6,688 nM. Compound (2b), as single agent, inhibited the proliferation of UACC-62 cells with an IC40 of 35 nM (Table 11).

***Table 11: Absolute $IC_{40}$ estimations for each compound alone in example 4***

| Absolute $IC_{40}$ of single agents are estimated with 4-parameter logistic models | |
|---|---|
| | Absolute IC40s (nM) |
| Compound (I) | 6687.6 [1809.3; 11566] |
| Compound (2b) | 34.9 [28.6; 41.3] |

As described by the isobologram representation in Figure 4, in the combination rays, synergy was observed at equipotent concentrations (Ray 4 bis (f=0.62)) and at concentration so that compound (2b) was more effective than compound (I) (ratio 1/19 (f=0.05): Ray 2, ratio 1/6 (f=0.14): Ray 3 and Ratio ½ (f=0.34): Ray 4), with Ki of 0.36 [0.17-0.54], 0.55 [0.42-0.69], 0.28 [0.19-0.37], and 0.33 [0.20-0.45] respectively (Table 12).

***Table 12: Interaction characterization in example 4***

| Interaction indexes (Ki) allow us to define the interaction observed between the two compounds. | | | |
|---|---|---|---|
| | **f values** | **Ki (confidence interval at 95%)** | **Interaction characterization** |
| **Ray 2** | 0.05 | 0.5545 [0.4155; 0.6936] | Synergy |
| **Ray 3** | 0.14 | 0.2795 [0.1923; 0.3668] | Synergy |
| **Ray 4** | 0.34 | 0.3279 [0.2033; 0.4526] | Synergy |
| **Ray 4bis** | 0.62 | 0.3562 [0.1693; 0.5431] | Synergy |

In the studied domain, synergy is observed when f is equal to 0.05, 0.14, 0.34 and 0.62.

These data correspond to a representative study out of 3 independent experiments. For these three experiments, synergy or additivity with tendency to synergy was observed for an effective fraction f between 0.05 and 0.62.

Summary of *in vitro* results (example 1 to 4)

By the above data, it is demonstrated that a selective PI3Kβ inhibitor (compound I) can synergize with MEK inhibitors (compound 2a) and with RAF inhibitors (compound 2b) to increase the inhibitory activity on cell proliferation in tumor indications exhibiting PI3Kβ pathway activation through PTEN deficiency and MAPK pathway activation, in particular through BRAF activating mutations.

**Example 5**: *In vivo* activity of compound (I) in combination with compound (2a) against subcutaneous human melanoma tumors UACC-62 bearing SCID female mice

To evaluate the antitumor activity of the PI3Kβ selective inhibitor compound (I) in combination with the MEK inhibitor compound (2a), experiments were conducted using female SCID mice bearing human melanoma tumors UACC-62 (BRAF mutant and PTEN-deficient). In the study, compound (I) at 150 mg/kg bi daily (bid) was tested in

combination with compound (2a) at 10 and 25 mg/kg daily (qd)

Materials and methods

CB17/ICR-Prkdc severe combined immunodeficiency (SCID)/Crl mice, at 8-10 weeks old, were bred at Charles River France (Domaine des Oncins, 69210 L'Arbresle, France) from strains obtained from Charles River, USA. Nude NIH-Foxn1 RNU/Crl rats, at 4-5 weeks old, were bred at Charles River USA (Wilmington, MA, USA). Mice and rats were over 18 g and 100 g, respectively, at start of treatment after an acclimatization time of at least 5 days. They had free access to food (UAR reference 113, Villemoisson, 91160 Epinay sur Orge, France) and sterile water. They were housed on a 12 hours light/dark cycle. Environmental conditions including animal maintenance, room temperature (22°C ± 2°C), relative humidity (55% ± 15%) and lighting times were recorded by the supervisor of laboratory animal sciences and welfare (LASW) and the records are archived.

The human melanoma UACC-62 tumor model was established by implanting subcutaneously (SC) $3 \times 10^6$ cells mixed with 50% matrigel per SCID female mice.

Compound (I) formulation was prepared in solution in 12.5% Ethanol / 12.5% Polysorbate 80 / 75% Isotonic glucose 5% in water pH 2. The preparation was stored in the dark at room temperature (RT). The stock solution was chemically stable 7 days. The volume of per os (PO) administration per mouse was 10 mL/kg.

Compound (2a) formulation was prepared in 0.5% hydroxy propyl methyl cellulose / 0.1% Polysorbate 80 in water. The stock solution was chemically stable 7 days in the dark at RT and resuspended before dosing. The volume of PO administration per mouse was 10 mL/kg.

For subcutaneous implantation of tumor cells, skin in the flank of the mice was disinfected using alcohol or Betadine® solution (Alcyon) and a suspension of tumor cells was inoculated SC unilaterally under a volume of 0.2 mL using a 23 G needle.

The dosages and schedule of administration of compound (I) and compound (2a) and compound (2b) used as single agent or in combination are described in the results section and detailed in the below tables 13 to 15.

The animals required to begin a given experiment were pooled and implanted monolaterally on day 0. Treatments were administered on measurable tumors. The solid tumors were allowed to grow to the desired volume range (animals with tumors not in the desired range were excluded). The mice were then pooled and unselectively distributed to the various treatment and control groups. Treatment started 11 days post UACC-62 cell tumor implantation as indicated in the results section and in each table. The dosages are expressed in mg/kg, based on the body weight at start of therapy. Mice were checked daily, and adverse clinical reactions noted. Each group of mice was weighed as a whole daily until the weight nadir was reached. Then, groups were weighed once to thrice weekly until the end of the experiment. Tumors were measured with a caliper 2 to 3 times weekly until final sacrifice for sampling time, tumor reached 2000 $mm^3$ or until the animal died (whichever comes first). Solid tumor volumes were estimated from two-dimensional tumor measurements and calculated according to the following equation:

$$\text{Tumor volume } (mm^3) = \text{Length } (mm) \times \text{Width}^2 \, (mm^2)/2$$

The day of death was recorded. Surviving animals were sacrificed and macroscopic examination of the thoracic and abdominal cavities was performed.

A dosage producing a 15% body weight loss (bwl) during three consecutive days (mean of group), 20% bwl during 1 day or 10% or more drug deaths was considered an excessively toxic dosage. Animal body weights included the tumor weight.

The primary efficacy end point is tumor volume changes from baseline summarized by the ratio of medians between treated and control groups ($\Delta T/\Delta C$).

Changes in tumor volume for each treated (T) and control (C) group are calculated for each animal and each day by subtracting the tumor volume on the day of first treatment (staging day) from the tumor volume on the specified observation day. The median $\Delta T$ is calculated for the treated group and the median $\Delta C$ is calculated for the control group. Then the ratio $\Delta T/\Delta C$ is calculated and expressed as a percentage:

$$\Delta T/\Delta C = (\text{median delta T/ median delta C}) \times 100$$

In this model the dose is considered statistically significant when $\Delta T/\Delta C$ is lower than 40%.

The term "therapeutic synergy" is used when the combination of two products at given doses is more efficacious than the best of the two products alone considering the same doses. In order to study therapeutic synergy, a Dunnett's

test to compare each combination to both single agents at the dose involved in the combination were performed after a two-way analysis of variance on rank-transformed tumor volume changes from baseline. Statistical analyses were performed on SAS system release 8.2 for SUN4 via Everstat V5 software and SAS 9.2 software. A probability less than 5% ($p<0.05$) was considered as significant.

Results of *in vivo* studies

The median tumor burden at start of therapy was 126 to 144 $mm^3$. As single agents, compound (I) (150 mg/kg/adm) and compound (2a) (25 and 10 mg/kg/adm) were administered PO bid and qd, respectively, from days 11 to 22 post tumor implantation. In the combination groups, the dose of compound (I) was combined with each dose of compound (2a) as shown in Table 13.

Compound (I) and compound (2a) as single agents or used in combination were well tolerated inducing minimal bwl (Figure 5 and Table 13).

As single agent, compound (I) (150 mg/kg, bid) was not significantly significant ΔT/ΔC > 40%. compound (2a) at 25 mg/kg qd was active (ΔT/ΔC = 2% on day 22) and the dose level below at 10 mg/kg qd was active (ΔT/ΔC = 35% on day 22) under these test conditions (Figure 6 and Table 13).

In the combination, the treatment with compound (I) and compound (2a) at 25 and 10 mg/kg qd were active (both with a ΔT/ΔC < 0% on day 22) (Figure 6 and Table 13). As shown by Table 14, therapeutic synergy was reached for both combinations for global analysis. *See also* Table 15.

***Table 13: Antitumor activity of compound (I) in combination with compound (2a) against human UACC-62 bearing SCID female mice***

| Agent (batch) | Route/ Dosage in mL/kg per injection | Dosage in mg/kg per injection (total dose) | Schedule in days | Drug death (Day of death) | Average body weight change in % per mouse at nadir (day of nadir) | ΔT/ΔC % (day 22) |
|---|---|---|---|---|---|---|
| Cpd. I ((VAC.XF Q6.183.1 ) | PO 10 m L/kg | 150 bid [a] (3450) | 11-22 | 0/7 | -4.6 (19) | 72 |
| Cpd. 2a (VAC. HA L1.179) | PO 10 m L/kg | 25 (300) | 11-22 | 0/7 | -6.2 (17) | 2 |
| | | 10(120) | | 0/7 | -4.3 (21) | 35 |
| Cpd. I Cpd. 2a | PO 10 m L/kg | 150 bid [a] (3450) 25 (300) | 11-22 | 0/7 | -7.0 (17) | -7 |
| | | 150 bid [a] (3450) 10(120) | | 0/7 | -3.6 (17) | -2 |
| Control | | | | | -9.0 (22) | - |

Tumor size at start of therapy was 100-256 $mm^3$, with a median tumor burden per group of 144 $mm^3$. Drug formulation: Compound (I)= Ethanol/ Polysorbate 80/ Glucose 5 % in water (12.5/12.5/75); AZD-6244 = 0.5% hydroxyl propyl methyl cellulose / 0.1% PS80 in water. Treatment duration: 12 days. Abbreviations used: bid = bi daily treatment, HDT = highest dose tested,

Δ T/ΔC=Ratio of change in tumor volume from baseline median between treated and control groups (TVday - TV0) / (CVday - CV0) * 100.

[a] Compound (I): one administration on day 22

***Table 14: Antitumor activity of compound (I) in combination with compound (2a) against human UACC-62 bearing SCID female mice mice: Therapeutic synergy determination***

| **Tumor volume changes from baseline: Median (nMad) and Anova followed by a Dunnett's test on rank-transformed tumor volume changes from baseline** | | | | | | |
|---|---|---|---|---|---|---|
| Group | Day | | | | | |
| | Global | 13 | 15 | 18 | 20 | 22 |
| Cpd (I) 150 mg/kg bid + Cpd (2a) 25 mg/kg qd | -51 (28.2) - | -51 (19.3) - | -62 (23.7) - | -51 (51.9) - | -51 (28.2) - | -51 (54.9) |
| Cpd (I) 150 mg/kg bid | 226 (194.2) <.0001 | 51 (65.2) 0.0006 | 126(41.5) <.0001 | 194 (75.6) <.0001 | 308 (100.8) <.0001 | 524 (83) <.0001 |
| Cpd (2a) 25 mg/kg qd | 0 (53.4) 0.0010 | 0 (38.5) 0.2197 | 18 (62.3) 0.0015 | 0 (29.7) 0.0267 | 36 (59.3) 0.0001 | 18 (120.1) 0.0021 |
| Cpd (I) 150 mg/kg bid + Cpd (2a) 10 mg/kg qd | -20 (47.4) - | -20 (46) - | -46 (41.5) - | -51 (48.7) - | -16 (50.4) - | -16 (65.2) |
| Cpd (I) 150 mg/kg bid | 226 (194.2) <.0001 | 51 (65.2) 0.0020 | 126(41.5) <.0001 | 194 (75.6) <.0001 | 308 (100.8) <.0001 | 524 (83) <.0001 |
| Cpd (2a) 10 mg/kg qd | 101 (146.8) <.0001 | 0 (26.7) 0.0394 | 46 (14.8) 0.0083 | 101 (46) <.0001 | 180 (117.1) <.0001 | 252 (74.1) <.0001 |
| p-value: obtained with Dunnett's test to compare each combination to both single agents at the dose involved in the combination after 2-way Anova with repeated measures on rank-transformed tumor volume changes from baseline | | | | | | |

***Table 15:***

| | ΔT/ΔC (%) on d22 |
|---|---|
| Cpd. (I) 150 mg/kg bid | 72 |
| Cpd. (2a) 25 mg/kg qd | 2 |
| Cpd. (2a) 10 mg/kg qd | 35 |
| Cpd. (I) 150 mg/kg bid Cpd. (2a) 25 mg/kg qd | -7 |
| Cpd. (I) 150 mg/kg bid Cpd. (2a) 10 mg/kg qd | -2 |

Example 6: <u>*In vivo* activity of compound (I) in combination with compound (2b) against subcutaneous human melanoma tumors UACC-62 bearing SCID female mice</u>

To evaluate the antitumor activity of the PI3Kβ selective inhibitor compound (I) in combination with the RAF inhibitor compound (2b), experiments were conducted using female SCID mice bearing human melanoma tumors UACC-62 (BRAF mutant and PTEN-deficient). In the study, compound (I) at 151.5 mg/kg bi daily (bid) was tested in combination with compound (2b) at 50 and 100 mg/kg daily (qd).

Materials and methods

The human melanoma UACC-62 tumor model was established by implanting subcutaneously (SC) $3x10^6$ cells mixed with 50% matrigel per SCID female mice.

Compound (I) formulation was prepared according to the material and methods of example 5.

Compound (2b) formulation was prepared in 90 % Klucel 2% in water pH4 followed by vortexing and magnetic stirring. The pH of the final solution was 4 (yellow suspension). The stock solution was chemically stable 7 days in the dark at RT. The volume of PO administration per mouse was 10 mL/kg.

The dosages and schedule of administration of compound (I) and compound (2b) used as single agent or in combination are described in the results section and detailed in the tables that follow.

Treatment started 8 days post UACC-62 cell tumor implantation as indicated in the results section and in the tables below 16 to 18.

Materials and methods used here for animal husbanding, subcutaneous implantation of tumor cells, study monitoring, tumor volume, animal death and animal body weight loss are the same described in example 5.

The primary efficacy end points used are the same used in example 5.

Results of *in vivo* studies

The median tumor burden at start of therapy was 125 to 126 $mm^3$. As single agents, compound (I) (151.5 mg/kg/adm) and compound (2b) (100 and 50 mg/kg/adm) were administered PO bid and qd, respectively, from days 8 to 15 post tumor implantation. In the combination groups, the dose of compound (I) was combined with each dose of compound (2b) as shown in Table 16.

Compound (I) and compound (2b) as single agents or used in combination were well tolerated inducing minimal bwl (Figure 7 and Table 16).

As single agent, compound (I) (151.5 mg/kg, bid) was active (ΔT/ΔC = 39 on day 15). Compound (2b) at 100 mg/kg qd was active (ΔT/ΔC = 20 on day 15) and the dose level below at 50 mg/kg qd was active (ΔT/ΔC = 31 on day 15) under these test conditions (Figure 8 and Table 16).

In the combination, the treatment with compound (I) and compound (2b) at 100 and 50 mg/kg qd were active (ΔT/ΔC = 2 on day 15 and ΔT/ΔC = 11 on day 15, respectively) (Figure 8 and Table 16). As shown by Table 17, therapeutic synergy was reached for both combinations for global analysis. *See also* Table 18.

***Table 16: Antitumor activity of compound (I) in combination with compound (2b) against human UACC-62 bearing SCID female mice***

| Agent (batch) | Route/ Dosage in mL/kg per injection | Dosage in mg/kg per injection (total dose) | Schedule in days | Drug death (Day of death) | Average body weight change in % per mouse at nadir (day of nadir) | ΔT/ΔC % (day 15) |
|---|---|---|---|---|---|---|
| Cpd. I (VAC.XFQ6.183.1) | P.O 10 mL/Kg | 151.5 bid [b] (2272.5) | 8-15[a] | 0/7 | -2.9 (15) | 39 |
| Cpd. 2b (VAC.SON5.145) | P.O 10 mL/Kg | 100 (800) | 8-15 | 0/7 | -3.7 (13) | 20 |
| | | 50 (400) | | 0/7 | -1.7 (15) | 31 |
| Cpd. I Cpd. 2b | P.O 10 mL/Kg | 151.5 bid [b] (2272.5) 100 (800) | 8-15 [a] | 0/7 | -1.4 (15) | 2 |
| | | 151.5 bid [b] (2272.5) 50 (400) | | 0/7 | -3.7 (15) | 11 |

(continued)

| Agent (batch) | Route/ Dosage in mL/kg per injection | Dosage in mg/kg per injection (total dose) | Schedule in days | Drug death (Day of death) | Average body weight change in % per mouse at nadir (day of nadir) | ΔT/ΔC % (day 15) |
|---|---|---|---|---|---|---|
| Control | | | | | -2.2 (15) | |

Tumor size at start of therapy was 80-320 $mm^3$, with a median tumor burden per group of 125-126 $mm^3$. Drug formulation: Compound I = Ethanol/ Polysorbate 80/ Glucose 5 % in water (12.5/12.5/75); Compound (2b) = Klucel 2% in water pH=4. Treatment duration: 8 days.
Abbreviations used: bid = bi daily treatment, ΔT/ΔC=Ratio of change in tumor volume from baseline median between treated and control groups (TVday - TV0) / (CVday - CV0) * 100
[a] Compound I: One administration on day 15
[b] Compound I dosing at 151.5 mg/kg instead of 150 mg/kg.

***Table 17 Antitumor activity of compound (I) in combination with compound (2b) against human UACC-62 bearing SCID female mice: Therapeutic synergy determination***

**Tumor volume changes from baseline: Median (nMad) and Anova followed by a Dunnett's test on rank-transformed tumor volume changes from baseline**

| Group | Day | | | |
|---|---|---|---|---|
| | Global | 11 | 13 | 15 |
| Compound I 151.5mg/kg bid + Compound 2b 1 00mg/kg qd | 18 (26.7)<br>- | 0 (46)<br>- | 18 (19.3)<br>- | 18 (72.6)<br>- |
| Compound I 151.5 mg/kg bid | 145 (89)<br><.0.0001 | 54 (62.3)<br>0.0366 | 145 (71.2)<br><.0.0001 | 303 (63.8)<br><.0.0001 |
| Compound 2b 100mg/kg qd Compound 2b 100mg/kg qd | 98 (89)<br>0.0104 | 54 (60.8)<br>0.1780 | 126 (74.1)<br>0.0173 | 152 (161.6)<br>0.0045 |
| Compound I 151.5mg/kg bid + compound 2b 50mg/kg qd | 12 (37.1)<br>- | -13 (17.8)<br>- | 12 (19.3)<br>- | 84 (108.2)<br>- |
| Compound I 151.5mg/kg bid | 145 (89)<br>0.0005 | 54 (62.3)<br>0.0270 | 145 (71.2)<br><.0001 | 303 (63.8)<br>0.0023 |
| Compound 2b 50mg/kg qd | 154 (136.4)<br><.0001 | 62 (16.3)<br>0.0019 | 157 (132)<br><.0001 | 240 (132)<br>0.0059 |

p-value: obtained with Dunnett's test to compare each combination to both single agents at the dose involved in the combination after 2-way Anova with repeated measures on rank-transformed tumor volume changes from baseline

***Table 18.***

| | ΔT/ΔC (%) on d15 |
|---|---|
| Cpd. I 151.5mg/kg bid | 39 |
| Cpd. 2b 100mg/kg qd | 20 |
| Cpd. 2b 50mg/kg qd | 31 |
| Cpd. I 151.5mg/kg bid<br>Cpd. 2b 100mg/kg qd | 2 |
| Cpd. I 151.5mg/kg bid<br>Cpd. 2b 50mg/kg qd | 11 |

**Example 7:** *In vivo* activity of compound (I) in combination with compounds (2a) and (2b) against subcutaneous human melanoma tumors WM-266.4 bearing SCID female mice

To evaluate the antitumor activity of the PI3Kβ selective inhibitor compound (I) in combination with the MEK inhibitor compound (2a) and the RAF inhibitor compound (2b), experiments were conducted using female SCID mice bearing human melanoma tumors WM-266.4 (BRAF mutant and PTEN-deficient). In the study, compound (I) at 150 mg/kg bi daily (bid) was tested in combination with compound (2a) at 10 and 25 mg/kg daily (qd) and compound (2b) at 50 and 100 mg/kg daily (qd).

Materials and methods

The human melanoma WM-266.4 tumor model was established by implanting subcutaneously (SC) $3x10^6$ cells mixed with 50% matrigel per SCID female mice.

Compound (I), compound (2a) and compound (2b) formulations were prepared according to the material and methods of examples 5 and 6.

The dosages and schedule of administration of compound (I) and compounds (2a) and (2b) used as single agent or in combination are described in the results section and detailed in the below tables 19 to 21.

Treatment started 21 days post WM-266.4 cell tumor implantation as indicated in the results section and in each table.

Materials and methods used here for animal husbanding, subcutaneous implantation of tumor cells, study monitoring, tumor volume, animal death and animal body weight loss are the same described in example 5.

The primary efficacy end points used are the same used in example 5.

Results of *in vivo* studies

The median tumor burden at start of therapy was 144 $mm^3$. As single agents, compound (I) (150 mg/kg/adm), compound (2a) (25 and 10 mg/kg/adm) and compound (2b) (100 and 50 mg/kg/adm) were administered PO bi daily for compound (I) and daily for compounds (2a) and (2b), from days 21 to 31 post tumor implantation. In the combination groups, the dose of compound (I) was combined with each dose of compound (2a) and compound (2b) as shown in Table 19.

***Table 19. Antitumor activity of compound (I) in combination with compounds (2a) and (2b) against human WM-266.4 bearing SCID female mice***

| **Agent (batch)** | **Route/ Dosage in mL/kg per injection** | **Dosage in mg/kg per injection (total dose)** | **Schedule in days** | **Drug death (Day of death)** | **Average body weight change in % per mouse at nadir (day of nadir)** | **ΔT/ ΔC % (day 31)** |
|---|---|---|---|---|---|---|
| Cpd. I (VAC.JRP2.132.1) | PO 10 mL/kg | 150 bid (3150) [a] | 21-31 | 0/7 | -8.2 (31) | 36 |

(continued)

| Agent (batch) | Route/ Dosage in mL/kg per injection | Dosage in mg/kg per injection (total dose) | Schedule in days | Drug death (Day of death) | Average body weight change in % per mouse at nadir (day of nadir) | ΔT/ ΔC % (day 31) |
|---|---|---|---|---|---|---|
| Cpd. 2a (VAC.HAL1.179) | PO 10 mL/kg | 25 (300) [b] | 21-31 | 0/7 | -9.6 (31) | 21 |
| | PO 10 mL/kg | 10 (120) [b] | | 0/7 | -10.5 (31) | 36 |
| Cpd. 2b (VAC.SON5.145) | PO 10 mL/kg | 100 (1100) | 21-31 | 0/7 | -9.4 (31) | 59 |
| | | 50 (550) | | 0/7 | -6.6 (31) | 59 |
| Cpd. I Cpd. 2a | PO 10 mL/kg | 150 bid (3150) [a] 25 (300) [b] | 21-31 | 0/7 | -11.3 (31) | 2 |
| | | 150 bid (3150) [a] 10 (120) [b] | | 0/7 | -13.0 (31) | 14 |
| Cpd. I Cpd. 2b | PO 10 mL/kg | 150 bid (3150) [a] 100 (1100) | 21-31 | 0/7 | -12.4 (28) | 30 |
| | | 150 bid (3150) [a] 50 (550) | | 0/7 | -9.9 (27) | 35 |
| Control | | | | | -8.3 (31) | |

Tumor size at start of therapy was 100-196 $mm^3$, with a median tumor burden per group of 144 $mm^3$. Drug formulation: Compound (I): Ethanol, Polysorbate 80, glucose 5% in water pH2 (12.5/12.5/75%), Compound (2b) = Klucel 2% pH=4, Compound (2a) = 0.5% hydroxyl propyl methyl cellulose / 0.1% PS80 in water. Treatment duration: 11 days. Abbreviations used: bid = bi daily treatment, ΔT/ΔC=Ratio of change in tumor volume from baseline median between treated and control groups (TVday - TV0) / (CVday - CV0) * 100.

[a] Compound (I) : One administration on day 31

[b] Compound (2a): 50mg/kg administered on day 21 instead of 25mg/kg and 20mg/kg administered on day 21 instead of 10mg/kg.

*Antitumor activity of compound (I) in combination with compound (2a) against human WM-266.4 bearing SCID female mice:*

As single agent, compound (I) was well tolerated as the bwl was comparable to the one induced by the tumor bearing control mice whereas compound (2a) induced a higher bwl as compared to the control. Compound (I) and compound (2a) used in combination were tolerated inducing a bwl comparable to the one induced by compound (2a) alone (Figure 9 and Table 19 above).

As single agent, compound (I) (150 mg/kg, bid) was active (ΔT/AC = 36 on day 31). Compound (2a) at 25 mg/kg qd was active (ΔT/ΔC = 21 on day 31) and the dose level below at 10 mg/kg qd was active (ΔT/ΔC = 36 on day 31) under these test conditions (Figure 10 and Table 19 above).

In the combination, the treatment with compound (I) and compound (2a) at 25 and 10 mg/kg qd were active (ΔT/ΔC = 2 on day 31 and ΔT/ΔC = 14 on day 31, respectively) (Figure 10 and Table 19 above). As shown by Table 20 below, therapeutic synergy was reached for both combinations for global analysis. *See also* Table 21 below.

*Antitumor activity of compound (I) in combination with compound (2b) against human WM-266.4 bearing SCID female mice:*

As single agent, compound (I) and compound (2b) were well tolerated as the bwl was comparable to the one induced by the tumor bearing control mice. Compound (I) and compound (2b) used in combination were tolerated inducing a bwl higher to the one induced by either of the single agents alone (Figure 11 and Table 19 above).

As single agent, compound (I) (150 mg/kg bid) was active (ΔT/ΔC = 36 on day 31). Compound (2b) at 100 and 50 mg/kg qd was not statiscally significant (ΔT/ΔC > 40 on day 31) under these test conditions (Figure 12 and Table 19 above).

In the combination, the treatment with compound (I) and compound (2b) at 100 and 50 mg/kg qd were active (ΔT/ΔC = 30 and 35 on day 31, respectively) (Figure 8 and Table 19 above). As shown by Table 20 below, therapeutic synergy was reached for the combinations of compound (I) with compound (2b) at 100 mg/kg qd for global analysis. *See also* Table 21 below.

***Table 20: Antitumor activity of compound (I) in combination with compounds (2a) and (2b) against human WM-266.4 bearing SCID female mice: Therapeutic synergy determination***

| Tumor volume changes from baseline: Median (nMad) and Anova followed by a Dunnett's test on rank-transformed tumor volume changes from baseline | | | | | |
|---|---|---|---|---|---|
| Group | | Day | | | |
| | Global | 24 | 27 | 29 | 31 |
| Cpd. I 150mg/kg bid+ Cpd. 2a 25 mg/kg qd | -13 (35.6)<br>- | -13 (19.3)<br>- | -36 (16.3)<br>- | -18 (50.4) | 18 (46) |
| Cpd. I 150 mg/kg bid | 138 (100.8)<br><0.0001 | 54 (53.4)<br>0.0088 | 132 (62.3)<br><0.0001 | 162 (47.4)<br><0.0001 | 272 (20.8)<br><0.0001 |
| Cpd. 2a 25 mg/kg qd | 101 (82.3)<br><0.0001 | 36 (16.3)<br>0.1041 | 101 (46)<br><0.0001 | 124 (80.1)<br><0.0001 | 156 (44.5)<br>0.0008 |
| Cpd. I 150mg/kg bid + Cpd. 2a 10 mg/kg qd | 24 (38.5)<br>- | 0 (26.7)<br>- | 0 (53.4)<br>- | 36 (23.7) | 101 (63.8) |
| Cpd. I 150 mg/kg bid | 138 (100.8)<br><0.0001 | 54 (53.4)<br>0.0682 | 132 (62.3)<br><0.0001 | 162 (47.4)<br><0.0001 | 272 (20.8)<br><0.0001 |
| Cpd. 2a 10 mg/kg qd | 194 (79.3)<br><0.0001 | 54 (43)<br>0.0126 | 194 (26.7)<br><0.0001 | 222 (20.8)<br><0.0001 | 268 (26.7)<br><0.0001 |
| Cpd. I 150mg/kg bid + Cpd. 2b 100 mg/kg qd | 71.5 (100.1)<br>- | 0 (0)<br>- | 25 (50.4)<br>- | 132 (46) | 226 (112.7) |
| Cpd. I 150 mg/kg bid | 138 (100.8)<br>0.0129 | 54 (53.4)<br>0.1392 | 132 (62.3)<br><0.0001 | 162 (47.4)<br>0.0816 | 272 (20.8)<br>0.6333 |
| Cpd. 2b 100 mg/kg qd | 193.5 (200.2)<br>0.0002 | 36 (26.7)<br>0.1685 | 171 (40)<br><0.0001 | 226 (86)<br>0.0006 | 441 (96.4)<br>0.0023 |
| Cpd. I 150mg/kg bid + Cpd. 2b 50 mg/kg qd | 138 (125.3)<br>- | 36 (23.7)<br>- | 72 (53.4)<br>- | 162 (44.5) | 258 (89) |
| Cpd. I 150 mg/kg bid | 138 (100.8)<br>0.7333 | 54 (53.4)<br>0.8912 | 132 (62.3)<br>0.1033 | 162 (47.4)<br>0.8524 | 272 (20.8)<br>0.7117 |

(continued)

| Tumor volume changes from baseline: Median (nMad) and Anova followed by a Dunnett's test on rank-transformed tumor volume changes from baseline | | | | | |
|---|---|---|---|---|---|
| Group | Day | | | | |
| | Global | 24 | 27 | 29 | 31 |
| Cpd. 2b 50 mg/kg qd | 274 (228.3) 0.0052 | 116 (11.9) 0.0671 | 254 (118.6) 0.0003 | 322 (94.9) 0.0215 | 442 (60.8) 0.3559 |
| p-value: obtained with Dunnett's test to compare each combination to both single agents at the dose involved in the combination after 2-way Anova with repeated measures on rank-transformed tumor volume changes from baseline | | | | | |

***Table 21***

| | ΔT/ΔC (%) on d31 |
|---|---|
| Cpd. I 150mg/kg bid | 36 |
| Cpd. 2a 25mg/kg qd | 21 |
| Cpd. 2a 10mg/kg qd | 36 |
| Cpd. 2b 100mg/kg qd | 59 |
| Cpd. 2b 50mg/kg qd | 59 |
| Cpd. I 150mg/kg bid Cpd. 2a 25mg/kg qd | 2 |
| Cpd. I 150mg/kg bid Cpd. 2a 10mg/kg qd | 14 |
| Cpd. I 150mg/kg bid Cpd. 2b 1 00mg/kg qd | 30 |
| Cpd. I 150mg/kg bid Cpd. 2b 50mg/kg qd | 35 |

**Example 8:** *In vivo* activity of compound (I) in combination with compound (2a) against subcutaneous human primary tumors CR-IGR-014P bearing SCID female mice

To evaluate the antitumor activity of the PI3Kβ selective inhibitor compound (I) in combination with the MEK inhibitor compound (2a), experiments were conducted using female SCID mice bearing human colon primary tumors CR-IGR-014P (KRAS mutant PTEN-deficient) xenografts. In the studies, compound (I) at 150 mg/kg bi daily (bid) was tested in combination with compound (2a) at 10 and 25 mg/kg daily (qd).

Materials and methods

The human primary colon carcinoma CR-IGR-014P tumor model was established by implanting subcutaneously (SC) small tumor fragments and was maintained in SCID female mice using serial passages.

Compounds (I) and (2a) formulation were prepared according to the material and methods of example 5.

The dosages and schedule of administration of compounds (I) and (2a) used as single agent or in combination are described in the results section and detailed in the below tables 22 to 24.

Treatment started 20 days post CR-IGR-014P tumor fragment implantation as indicated in the results section and in each table.

Materials and methods used here for animal husbanding, subcutaneous implantation of tumor cells, study monitoring, tumor volume, animal death and animal body weight loss are the same described in example 5.

The primary efficacy end points used are the same used in example 5.

Results of *in vivo* studies

The median tumor burden at start of therapy was 139 to 144 $mm^3$. As single agents, compound (I) (150 mg/kg/adm) and compound (2a) (25 and 10 mg/kg/adm) were administered PO bi daily and daily, respectively, from days 20 to 36 post tumor implantation. In the combination groups, the dose of compound (I) was combined with each

dose of compound (2a) as shown in below Table 22.

Compounds (I) and (2a) as single agents were well tolerated inducing minimal bwl, and a higher bwl occurred when the drugs were used in combination but was not toxic (Figure 13 and Table 22).

As single agents, compound (I) (150 mg/kg, bid) and compound (2a) (25 and 10 mg/kg qd) were not statistically significant (ΔT/ΔC > 40) under these test conditions (Figure 14 and below Table 22).

In the combination, the treatment with compound (I) and compound (2a) at 25 mg/kg qd was active (ΔT/ΔC = 28 on day 36) (Figure 14 and Table 22). As shown by Table 23, therapeutic synergy was reached for the combination of compound (I) with compound (2a) at 25 mg/kg qd for global analysis. *See also* Table 24 below.

***Table 22. Antitumor activity of compound (I) in combination with compound (2a) against human CR-IGR-014P bearing SCID female mice***

| **Agent (batch)** | **Route/ Dosage in mL/kg per injection** | **Dosage in mg/kg per injection (total dose)** | **Schedule in days** | **Drug death (Day of death)** | **Average body weight change in % per mouse at nadir (day of nadir)** | **ΔT/ΔC % (day 36)** |
|---|---|---|---|---|---|---|
| Cpd. I (VAC.JRP2.132.1) | P.O 10 mL/Kg | 150 bid (4950) [a] | 20-36 | 0/7 | -3.9 (27) | 94 |
| Cpd. 2a (VAC.HAL1.179) | P.O 10 mL/Kg | 25 (425) | 20-36 | 0/7 | -3.6 (33) | 45 |
| | | 10(170) | | 0/7 | -4.2 (27) | 84 |
| Cpd. I Cpd. 2a | P.O 10 mL/Kg | 150 bid (4950) a 25 (425) | 20-36 | 0/7 | -8.7 (31) | 28 |
| | | 150 bid (4950) a 10(170) | | 0/7 | -7.2 (31) | 76 |
| Control | | | | 0/7 | -3.8 (30) | |

Tumor size at start of therapy was 100-194 $mm^3$, with a median tumor burden per group of 139-144 $mm^3$. Drug formulation: Compound (I) = Ethanol/ Polysorbate 80/ Glucose 5 % in water (12.5/12.5/75); Compound (2a) = 0.5% hydroxyl propyl methyl cellulose / 0.1% PS80 in water. Treatment duration: 17 days. Abbreviations used: bid = bi daily treatment, ΔT/ΔC=Ratio of change in tumor volume from baseline median between treated and control groups (TVday - TV0) / (CVday - CV0) * 100.

[a] Compound (I): One administration on day 36

***Table 23. Antitumor activity of compound (I) in combination with compound (2a) against human CR-IGR-014P bearing SCID female mice: Therapeutic synergy determination***

| **Tumor volume changes from baseline: Median (nMad) and Anova followed by a Dunnett's test on rank-transformed tumor volume changes from baseline** | | | | | | | | |
|---|---|---|---|---|---|---|---|---|
| Group | Day | | | | | | | |
| | Global | 22 | 24 | 27 | 29 | 31 | 34 | 36 |
| Cpd. I 150mg/kg | - | 18 (19.3) | 18 (35.6) | 54 (19.3) | 157 (87.5) | 194 (81.5) | 304 (163.1) | 464 (148.3) |
| bid + cpd. 2a 10 mg/kg qd | - | - | - | - | - | - | - | - |

(continued)

**Tumor volume changes from baseline:<br>Median (nMad) and Anova followed by a Dunnett's test<br>on rank-transformed tumor volume changes from baseline**

| Group | Day | | | | | | | |
|---|---|---|---|---|---|---|---|---|
| | Global | 22 | 24 | 27 | 29 | 31 | 34 | 36 |
| Cpd. I 150 mg/kg bid | -<br>p=0.8335 | 24 (62.3)<br>p=0.9724 | 49 (72.6)<br>p=0.8793 | 132 (127.5)<br>p=0.2382 | 180 (106.7)<br>p=0.7411 | 274 (46)<br>p=0.8873 | 317 (164.6)<br>p=0.9295 | 572 (349.9)<br>p=0.7389 |
| cpd. 2a 10 mg/kg qd | -<br>p=0.5422 | 32 (20.8)<br>p=0.5678 | 52 (29.7)<br>p=0.0642 | 83 (56.3)<br>p=0.1217 | 190 (105.3)<br>p=0.9799 | 284 (152.7)<br>p=0.8523 | 275 (137.9)<br>p=0.9980 | 508 (152.7)<br>p=0.9971 |
| Cpd. I 150mg/kg bid + cpd. 2a 25 mg/kg qd | -<br>- | 0 (11.9)<br>- | -36 (8.9)<br>- | 0 (20.8)<br>- | 77 (32.6)<br>- | 144 (28.2)<br>- | 88 (38.5)<br>- | 168 (78.6)<br>- |
| Cpd. I 150 mg/kg bid | -<br>p=0.0007 | 24 (62.3)<br>p=0.1868 | 49 (72.6)<br>p=0.1260 | 132 180 (127.5)<br>p=0.0004 | (106.7)<br>p=0.1335 | 274 (46)<br>p=0.0219 | 317 (164.6)<br>p=0.0001 | 572 (349.9)<br>p<0.0001 |
| cpd. 2a 25 mg/kg qd | -<br>p=0.0197 | 12 (17.8)<br>p=0.3035 | 54 (41.5)<br>p=0.0293 | 54 (41.5)<br>p=0.0191 | 145 (17.8)<br>p=0.4477 | 194 145 194 170 (26.7)<br>p=0.1517 | 170 (71.2)<br>p=0.0371 | 272 (124.5)<br>p=0.0090 |

p-value: obtained with Dunnett's test to compare each combination to both single agents at the dose involved in the combination after 2-way Anova with repeated measures on rank-transformed tumor volume changes from baseline

***Table 24.***

| | ΔT/ΔC (%) on d36 |
|---|---|
| Cpd. I 150mg/kg bid | 94 |
| Cpd. 2a 25mg/kg qd | 45 |
| Cpd. 2a 10mg/kg qd | 84 |
| Cpd. I 150mg/kg bid<br>Cpd. 2a 25mg/kg qd | 28 |
| Cpd. I 150mg/kg bid<br>Cpd. 2a 10mg/kg qd | 76 |

<u>Summary of *in vivo* results (examples 5 to 8)</u>

When compound (I) was tested in combination with the MEK inhibitor compound (2a) and with the RAF inhibitor compound (2b) in the BRAF mutated and PTEN-deficient UACC-62 and WM-266.4 tumor models, the drugs used as single agents had some impact on tumor growth regardless of the dose used but the combination of the drugs was much more active inducing a sustained tumor stasis during the treatment phase and therapeutic synergy was reached. In the patient derived xenografts CR-IGR-014P harboring a KRas mutation and a PTEN deletion in which compound (I) has been combined with the MEK inhibitor compound (2a), a therapeutic synergy was also demonstrated.

Taken together, the selective PI3Kβ inhibitor compound (I) triggered a sustained antitumor activity when combined with targeted therapies such as MEK and RAF inhibitors in xenografts models with a dual PTEN deletion and a BRAF or a KRas mutation.

These *in vitro* and *in vivo* data support the benefit of using a PI3Kβ inhibitor, and in particular compound (I), in

combination with a MAPK pathway inhibitor as MEK inhibitors, and in particular compound (2a), or as RAF inhibitors, and in particular compound (2b), to treat tumors from different indications exhibiting PI3Kβ pathway activation through PTEN deficiency and MAPK pathway activation, in particular through BRAF activating mutations or RAS mutations. These tumors can be in particular melanoma PTEN-deficient/BRAF mutant.

By the above data it is demonstrated that:

- a selective PI13Kβ inhibitor (compound I) can synergize with MEK inhibitors (compound 2a) and with RAF inhibitors (compound 2b) to increase the inhibitory activity on cell proliferation in tumor indications exhibiting PI3Kβ pathway activation through PTEN deficiency and MAPK pathway activation, in particular through BRAF activating mutations.
- a selective PI3Kβ inhibitor (compound I) can synergize with MEK inhibitors (compound 2a) and with RAF inhibitors (compound 2b) to increase the anti-tumor activity without inducing added toxicity in preclinical animal models of tumor growth, in tumor indications exhibiting PI3Kβ pathway activation through PTEN deficiency and MAPK pathway activation, in particular through BRAF activating mutations.

## Claims

**1.** A pharmaceutical combination comprising:

- a compound of formula (I):

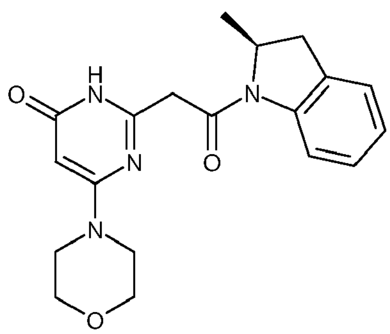

(I)

or a pharmaceutically acceptable salt thereof,
and
- at least one MAPK pathway inhibitor, or a pharmaceutically acceptable salt thereof.

**2.** The pharmaceutical combination of claim 1, wherein the MAPK pathway inhibitor is an inhibitor of one or both of a MEK kinase and a RAF kinase.

**3.** The pharmaceutical combination of claim 2, wherein the MAPK pathway inhibitor is a MEK inhibitor.

**4.** The pharmaceutical combination of claim 2, wherein the MAPK pathway inhibitor is a RAF inhibitor

**5.** The pharmaceutical combination of claim 2 , wherein the MAPK pathway inhibitor is:

- the compound (2a):

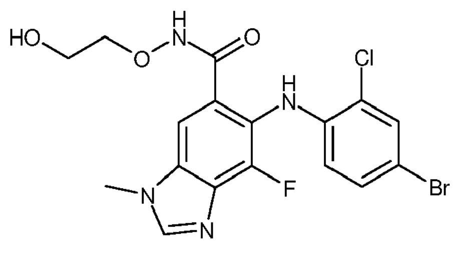

(2a)

or a pharmaceutically acceptable salt thereof,

or

- the compound (2b):

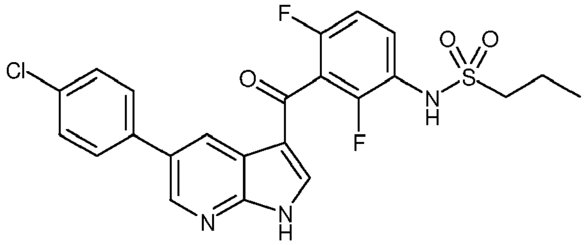

(2b)

or a pharmaceutically acceptable salt thereof.

**6.** The pharmaceutical combination of claim 2, wherein the MAPK pathway inhibitor is the compound (2a) of formula:

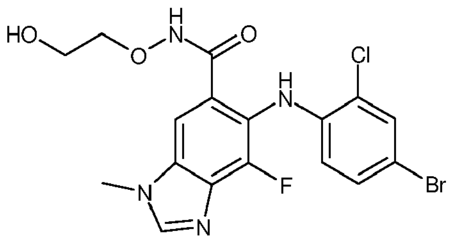

or a pharmaceutically acceptable salt thereof.

**7.** The pharmaceutical combination of claim 2, wherein the MAPK pathway inhibitor is the compound (2b) of formula:

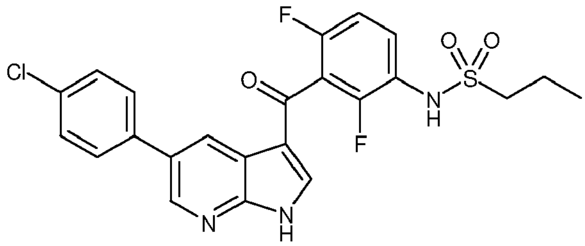

or a pharmaceutically acceptable salt thereof.

**8.** The pharmaceutical combination of any one of claims 1 to 7, further comprising a pharmaceutically acceptable carrier.

**9.** The pharmaceutical combination of any one of claims 1 to 7, comprising at least one further compound chosen from anticancer compounds.

**10.** A medicament comprising the pharmaceutical combination of any one of claims 1 to 9.

**11.** A pharmaceutical composition comprising the pharmaceutical combination of any one of claims 1 to 9, and a pharmaceutically acceptable excipient.

**12.** The pharmaceutical combination of any one of claims 1 to 9, for use as a medicament.

**13.** The pharmaceutical combination of any one of claims 1 to 9, for use for the treatment of cancer.

**14.** The combination according to claim 13, wherein the cancer is chosen from the group consisting of: non-small cell lung cancer, breast cancer, pancreatic cancer, liver cancer, prostate cancer, bladder cancer, cervical cancer, thyroid

cancer, colorectal cancer, liver cancer, muscle cancer, hematological malignancies, melanoma, endometrial cancer and pancreatic cancer.

**15.** The combination according to claim 13, wherein the cancer is chosen from the group consisting of: colorectal cancer, endometrial cancer, hematological malignancies, thyroid cancer, breast cancer, melanoma, pancreatic cancer and prostate cancer.

**16.** The combination according to claim 13, wherein administration of the compound of formula (I) is followed by the administration of the MAPK pathway inhibitor.

**17.** The combination according to claim 13, wherein administration of the MAPK pathway inhibitor is followed by the administration of the compound of formula (I).

**18.** The combination according to claim 13, wherein administration of the compound of formula (I) is followed by the administration of the compound (2a).

**19.** The combination according to claim 13, wherein administration of the compound of formula (I) is followed by the administration of the compound (2b).

**20.** The combination according to claim 13, wherein administration of the compound (2a) is followed by the administration of the compound of formula (I).

**21.** The combination according to claim 13, wherein administration of the compound (2b) is followed by the administration of the compound of formula (I).

**22.** The combination according to claim 13, wherein the compound of formula (I) and the MAPK pathway inhibitor are in amounts that produce a synergistic effect in reducing tumor volume.

**23.** The combination according to claim 13, wherein the compound of formula (I) and the MAPK pathway inhibitor are in amounts that produce a combined effect of tumor stasis.

**24.** The combination for its use according to claim 13, wherein the cancer is chosen from the group consisting of: non-small cell lung cancer, breast cancer, pancreatic cancer, liver cancer, prostate cancer, bladder cancer, cervical cancer, thyroid cancer, colorectal cancer, liver cancer, muscle cancer, hematological malignancies, melanoma, endometrial cancer and pancreatic cancer.

**25.** The combination for its use according to claim 13, wherein the cancer is chosen from the group consisting of: colorectal cancer, endometrial cancer, hematological malignancies, thyroid cancer, breast cancer, melanoma, pancreatic cancer and prostate cancer.

**26.** The combination for its use according to claim 13, by administration of the compound of formula (I) followed by the administration of the MAPK pathway inhibitor.

**27.** The combination for its use according to claim 13, by administration of the MAPK pathway inhibitor followed by the administration of the compound of formula (I).

**28.** The combination for its use according to claim 13, by administration of the compound of formula (I) followed by the administration of the compound (2a).

**29.** The combination for its use according to claim 13, by administration of the compound of formula (I) followed by the administration of the compound (2b).

**30.** The combination for its use according to claim 13, by administration of the compound (2a) followed by the administration of the compound of formula (I).

**31.** The combination for its use according to claim 13, by administration of the compound (2b) followed by the administration of the compound of formula (I).

**32.** The combination for its use according to claim 13, wherein the compound of formula (I) and the MAPK pathway inhibitor are in amounts that produce a synergistic effect in reducing tumor volume.

**33.** The combination for its use according to claim 13, wherein the compound of formula (I) and the MAPK pathway inhibitor are in amounts that produce a combined effect of tumor stasis.

**34.** A pharmaceutical combination comprising:

- a compound of formula (I):

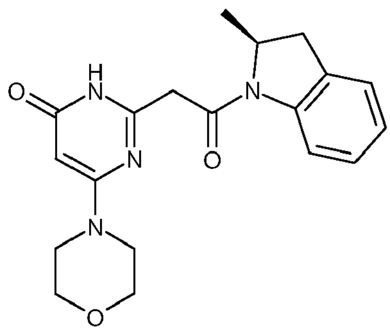

(I)

or a pharmaceutically acceptable salt thereof,
and at least one MAPK pathway inhibitor chosen from the group consisting of:

- the compound (2a):

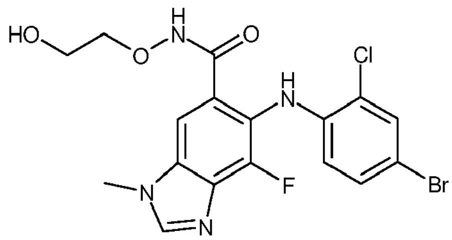

(2a)

or a pharmaceutically acceptable salt thereof,
and

- the compound (2b):

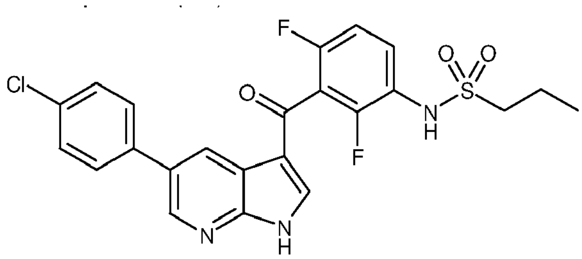

(2b)

or a pharmaceutically acceptable salt thereof, for use for the treatment of cancer.

**35.** A product comprising:

- a compound of formula (I):

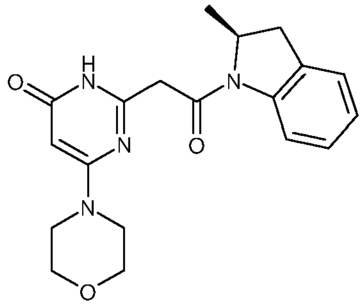

(I)

or a pharmaceutically acceptable salt thereof,
and
- at least one MAPK pathway inhibitor, or a pharmaceutically acceptable salt thereof,
as a combined preparation for simultaneous, separate or sequential use in anticancer therapy.

**36.** A kit comprising:

- at least one compound of formula (I):

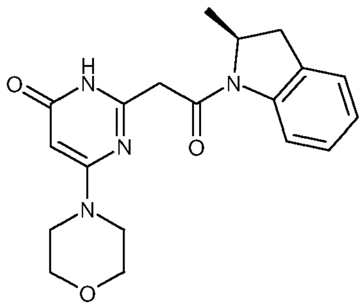

(I)

or a pharmaceutically acceptable salt thereof,
- at least one MAPK pathway inhibitor, or a pharmaceutically acceptable salt thereof,
and
- optionally, instructions for use.

**37.** A kit comprising:

- at least one compound of formula (I):

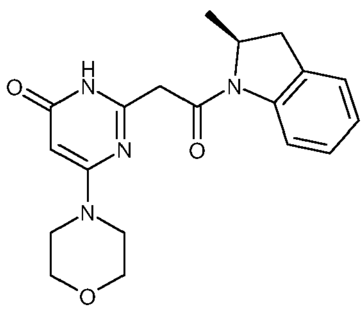

(I)

or a pharmaceutically acceptable salt thereof,
- at least one MAPK pathway inhibitor chosen from the group consisting of:

the compound (2a):

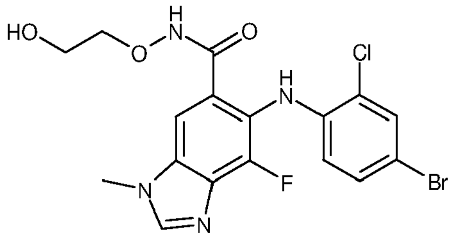

(2a)

or a pharmaceutically acceptable salt thereof, and

- the compound (2b):

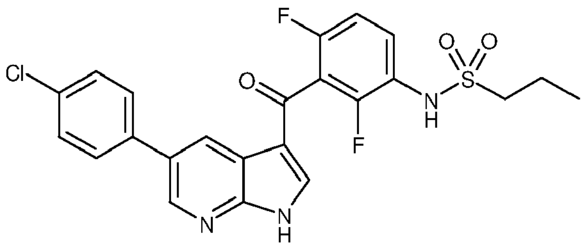

(2b)

or a pharmaceutically acceptable salt thereof,
- and optionally, instructions for use.

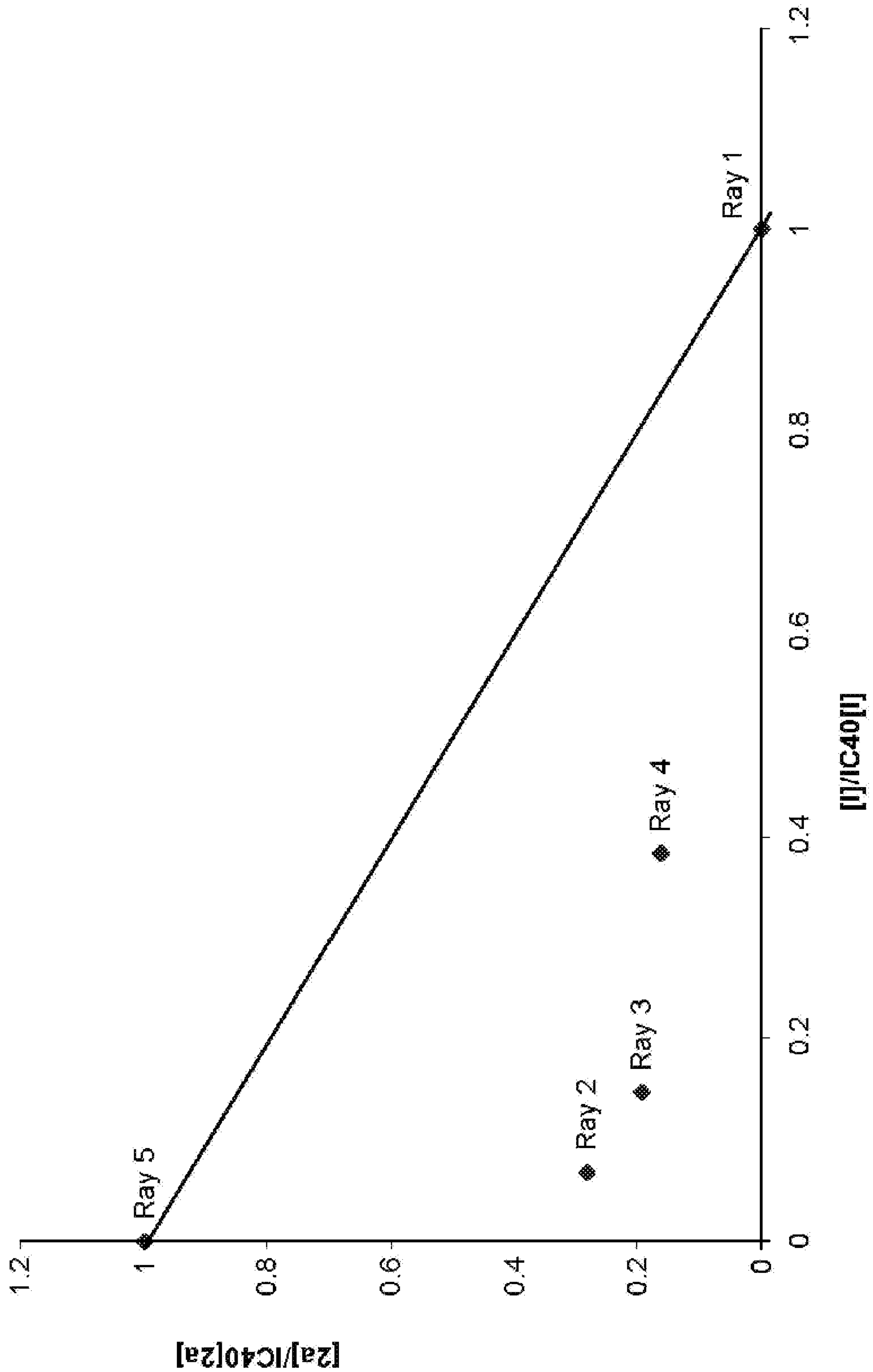


FIG.1

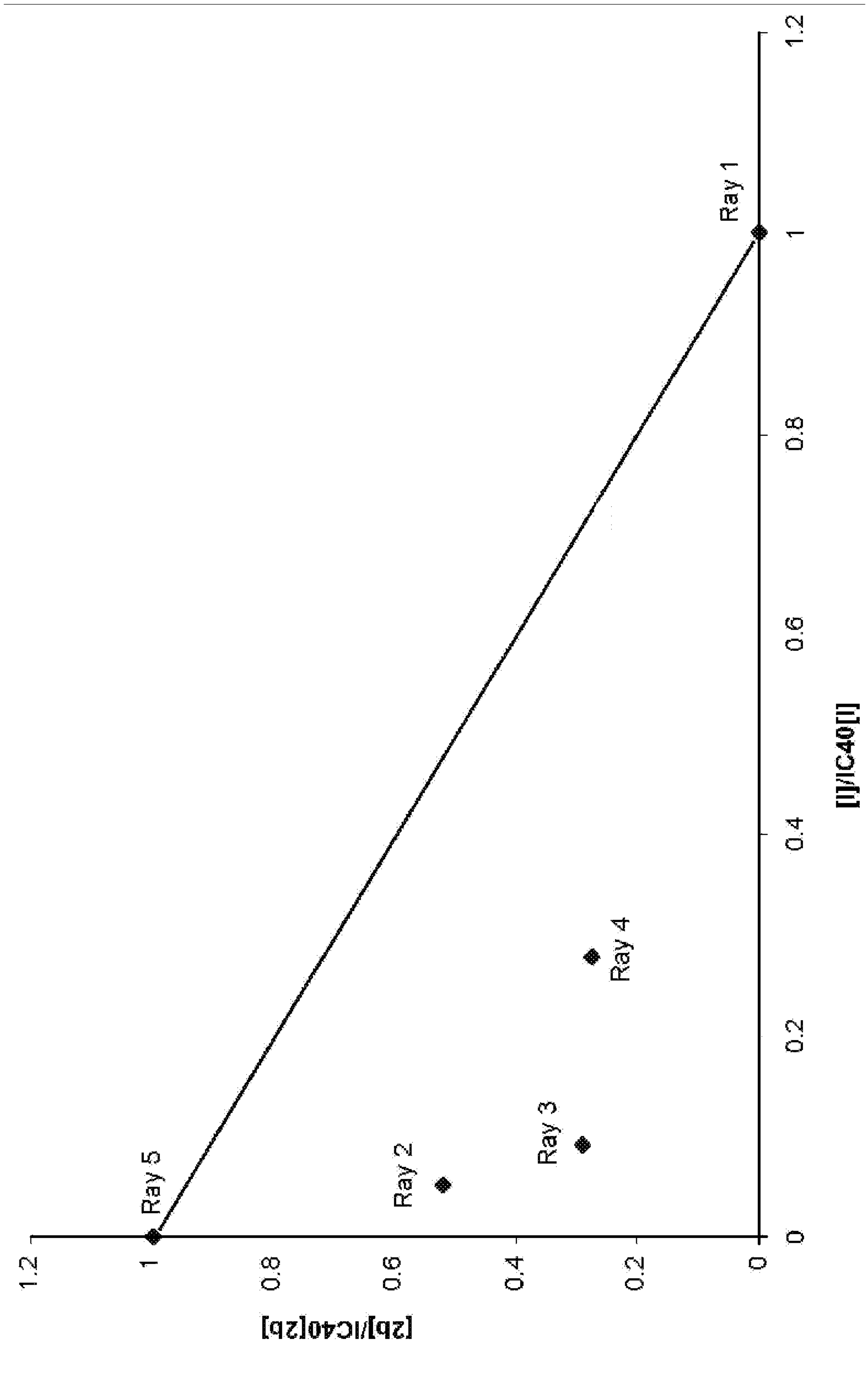


FIG.2

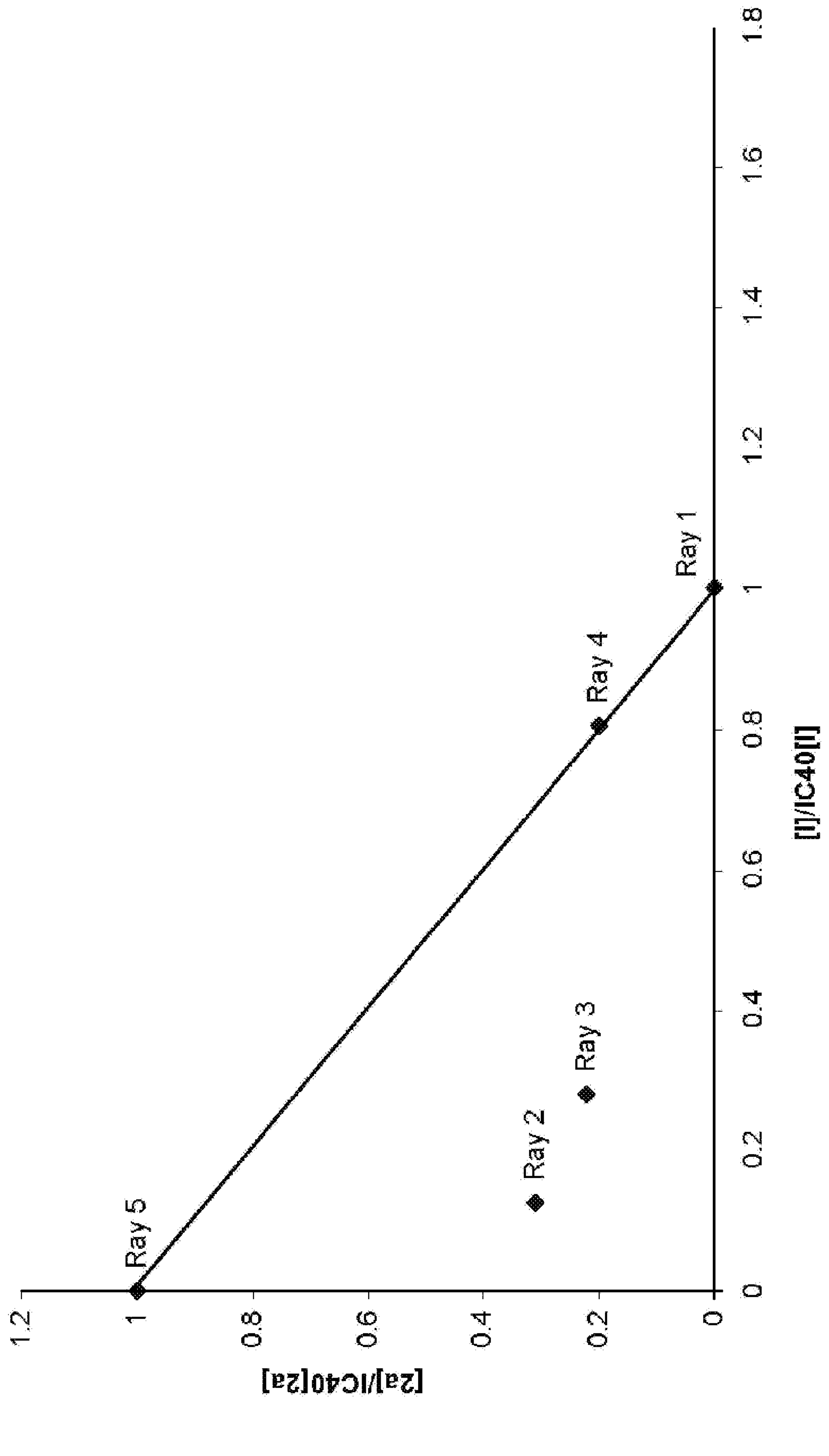
[2a]/IC40[2a]
[I]/IC40[I]
Ray 1
Ray 2
Ray 3
Ray 4
Ray 5
0
0.2
0.4
0.6
0.8
1
1.2
1.4
1.6
1.8

FIG.3

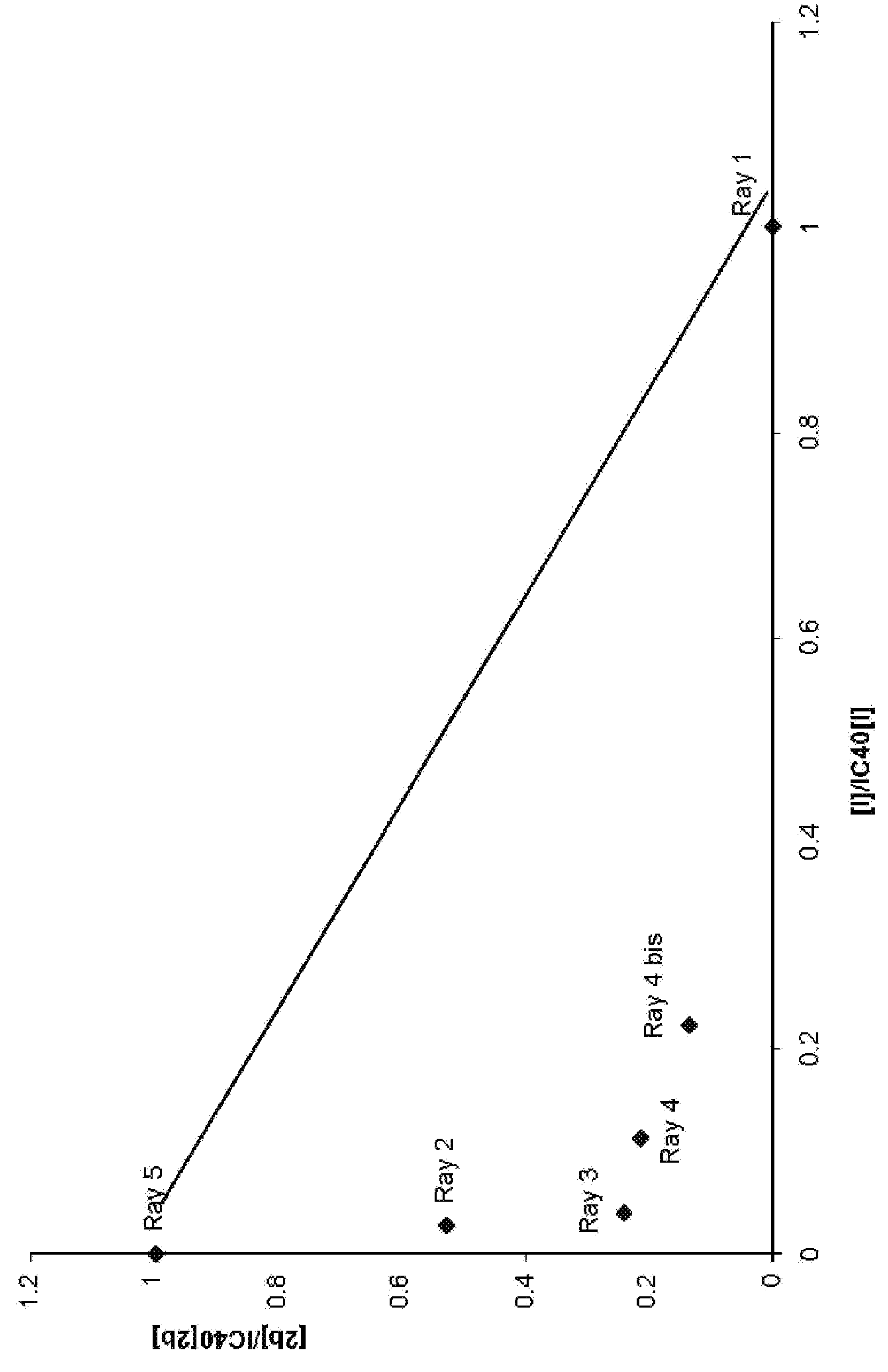

Ray 1
Ray 2
Ray 3
Ray 4
Ray 4 bis
Ray 5
[2b]/IC40[2b]
[I]/IC40[I]
0
0.2
0.4
0.6
0.8
1
1.2


FIG.4

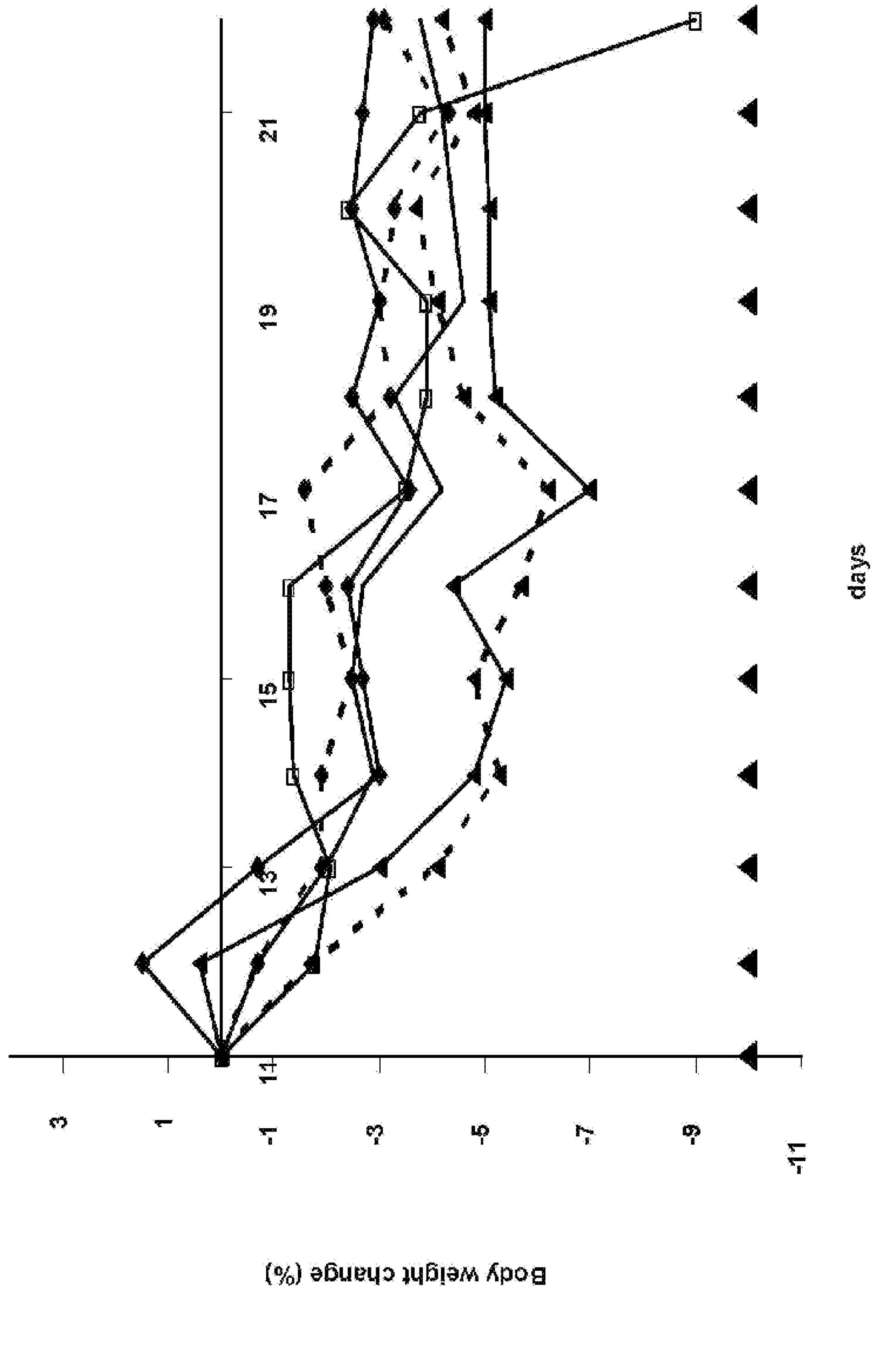


FIG.5

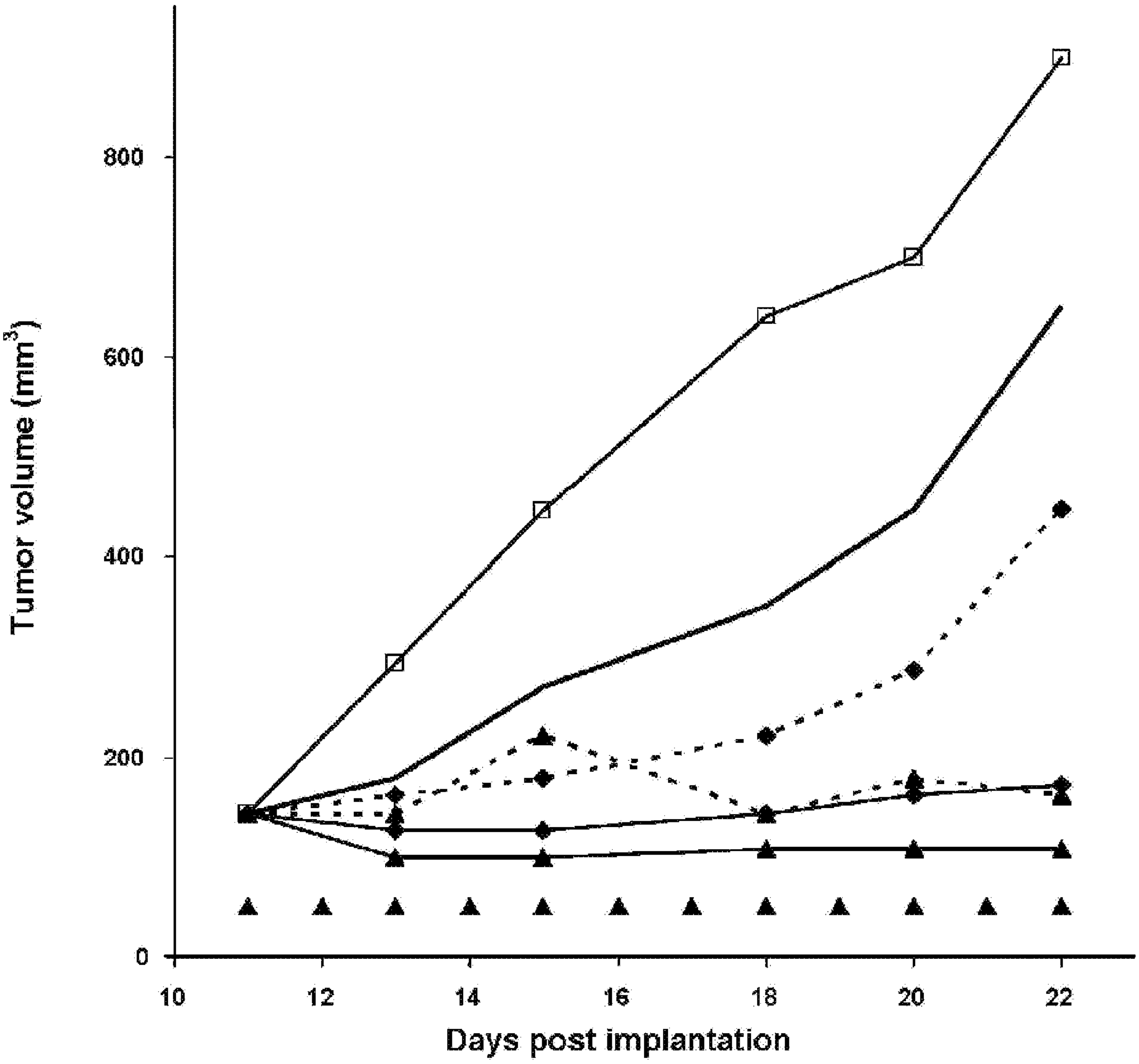
Tumor volume (mm3)
800
600
400
200
0
10
12
14
16
18
20
22
Days post implantation

FIG.6

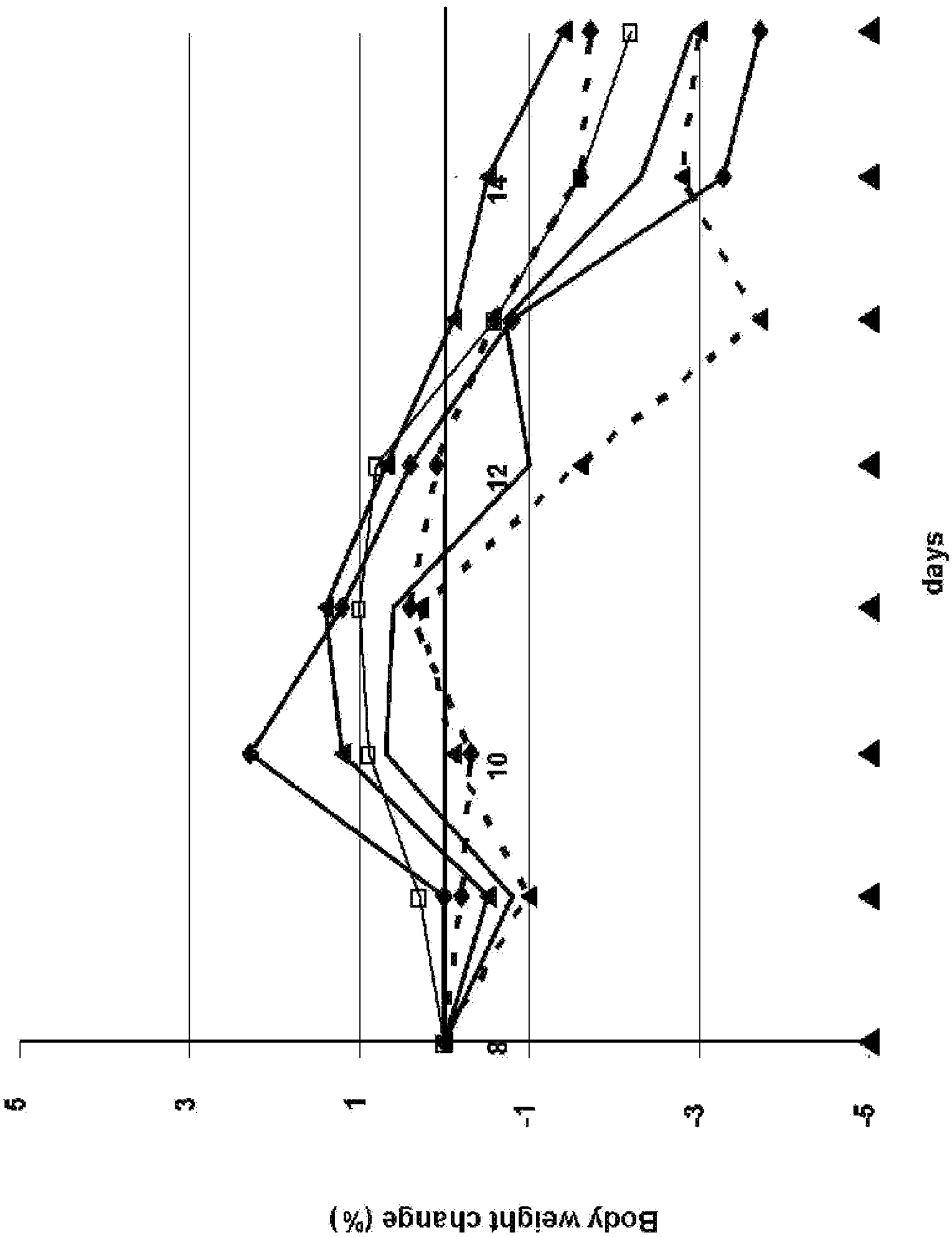


FIG.7

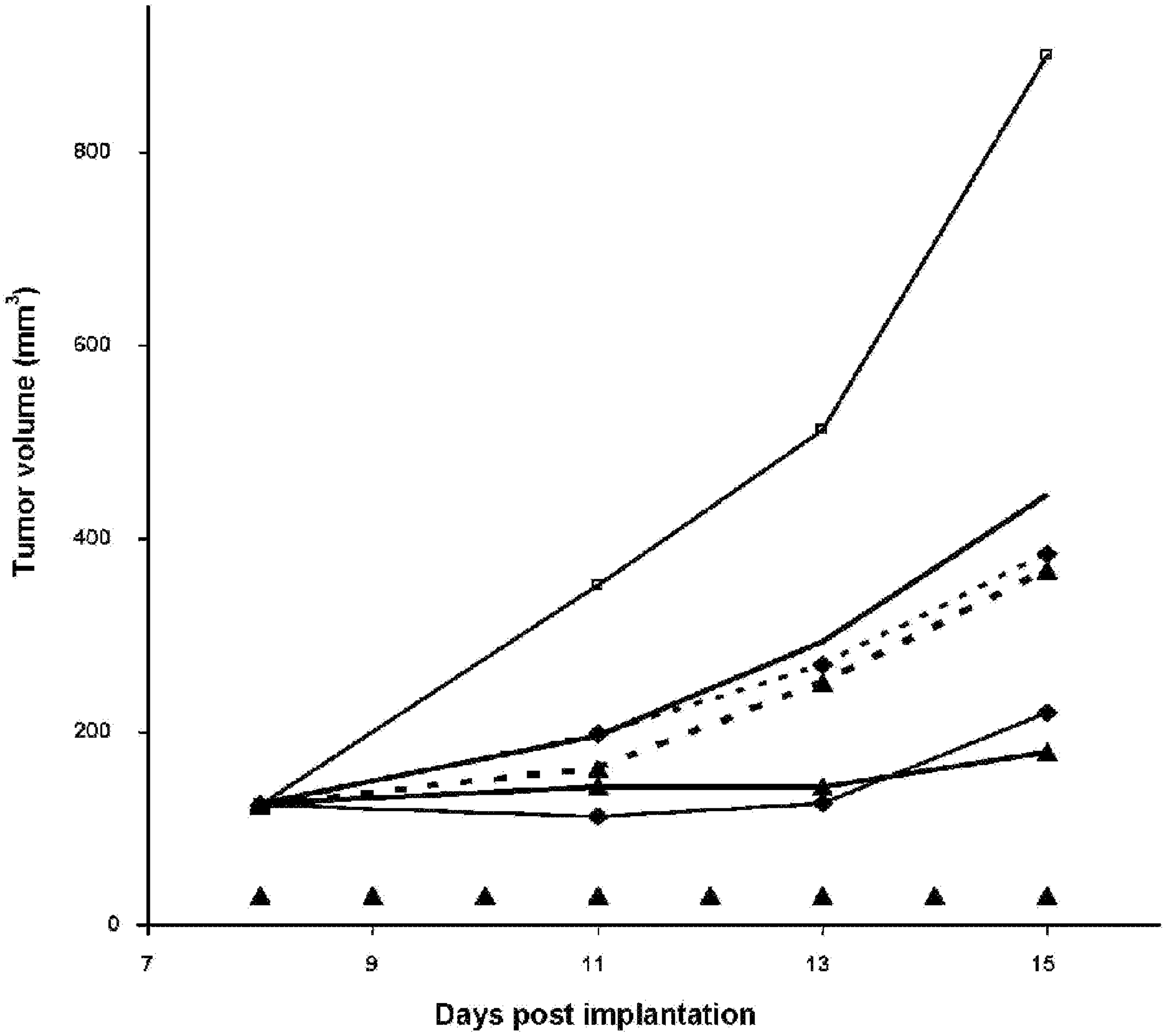
Tumor volume (mm3)
800
600
400
200
0
7
9
11
13
15
Days post implantation

FIG.8

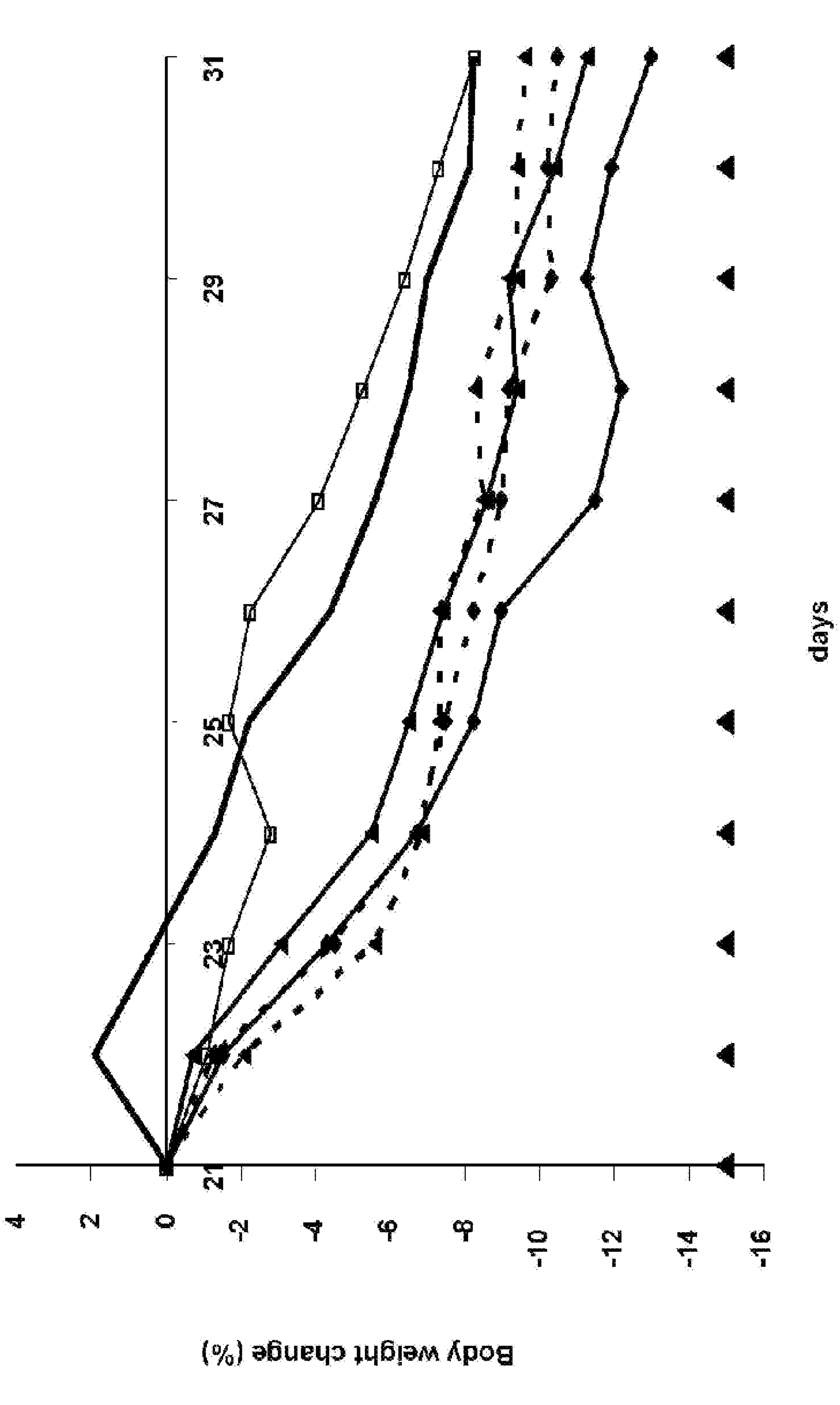


FIG.9

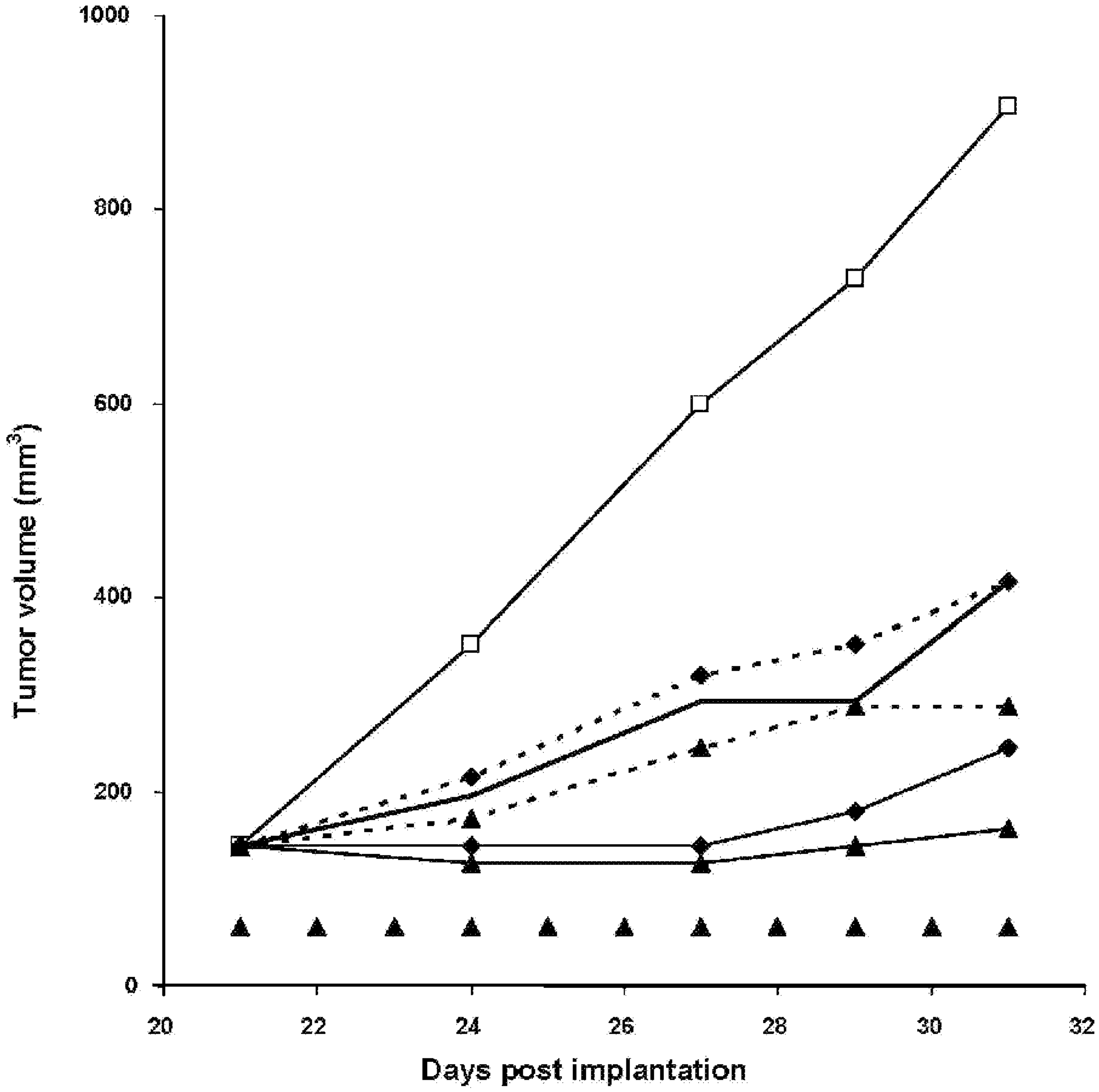
1000
800
600
400
200
0
Tumor volume (mm3)
20
22
24
26
28
30
32
Days post implantation

FIG.10

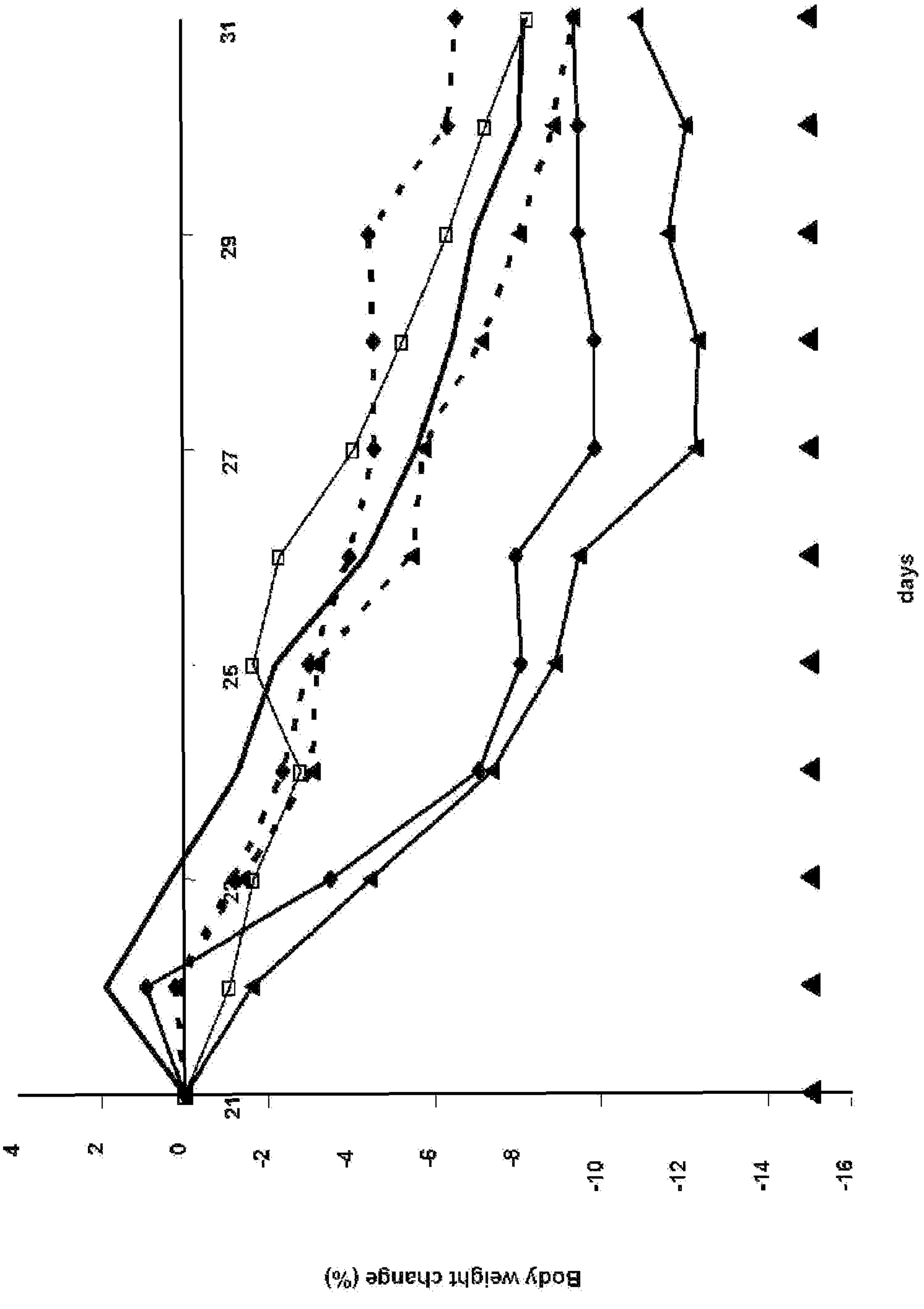


FIG.11

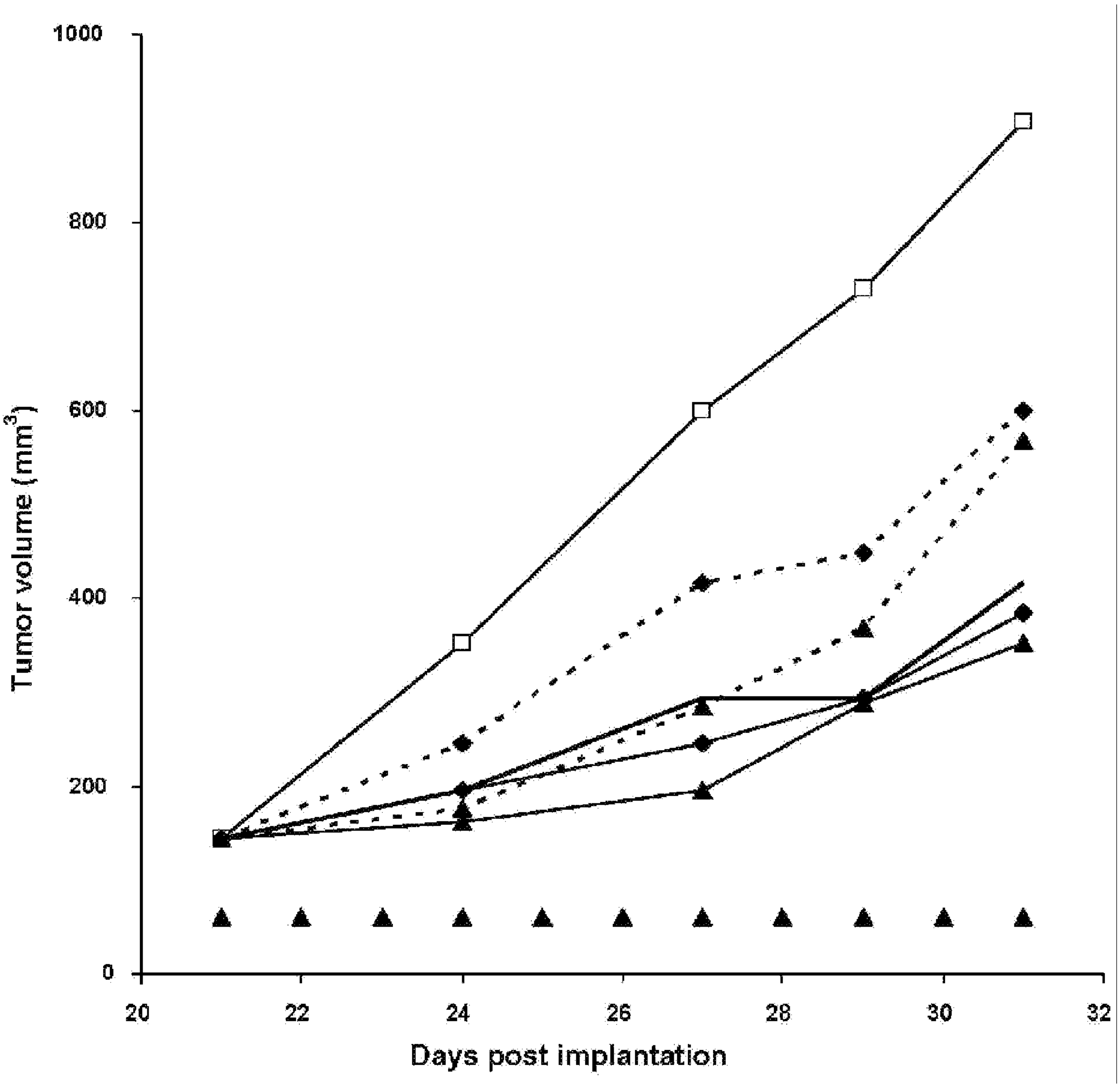
1000
800
600
400
200
0
Tumor volume (mm3)
20
22
24
26
28
30
32
Days post implantation

FIG.12

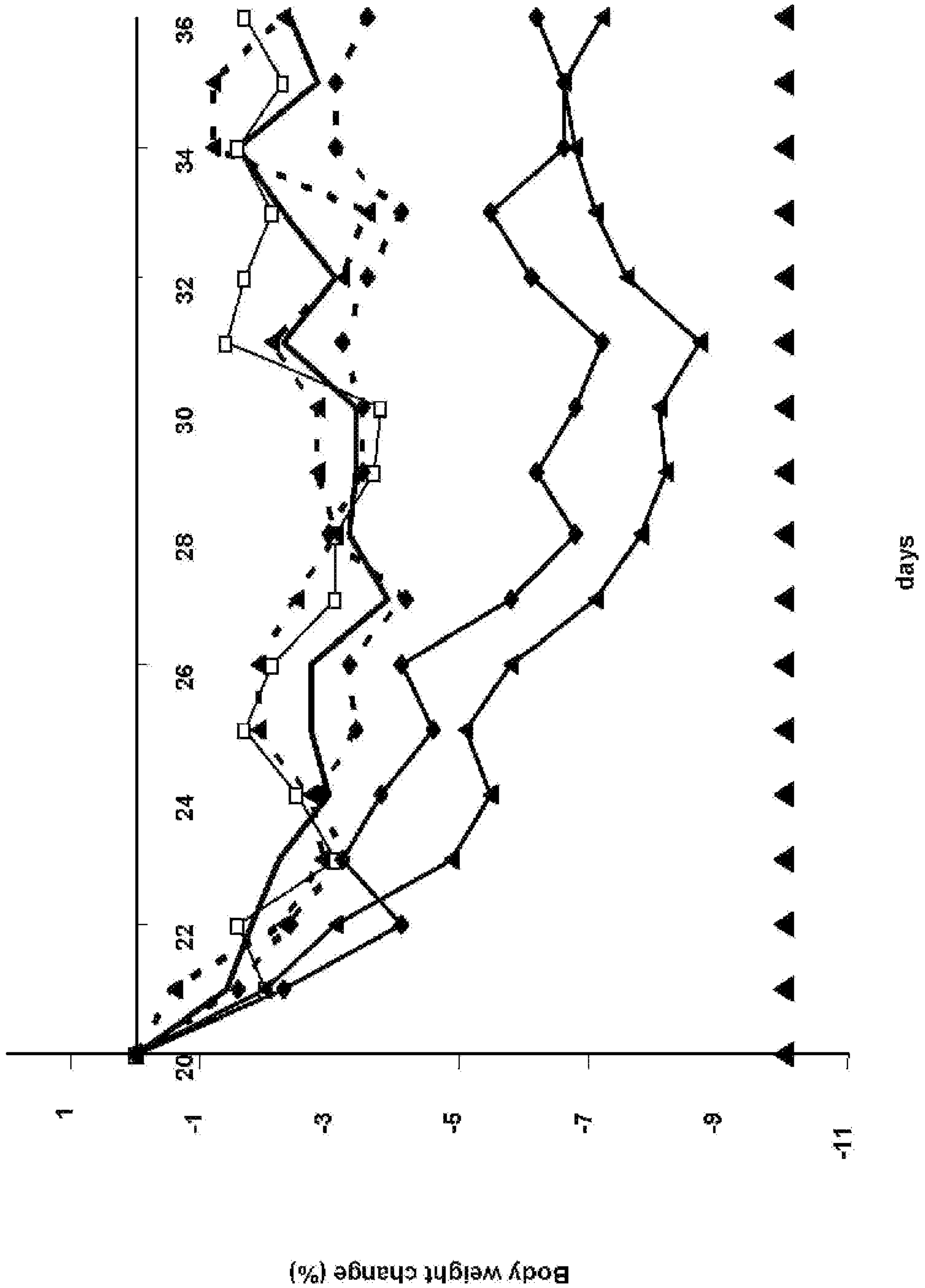


FIG.13

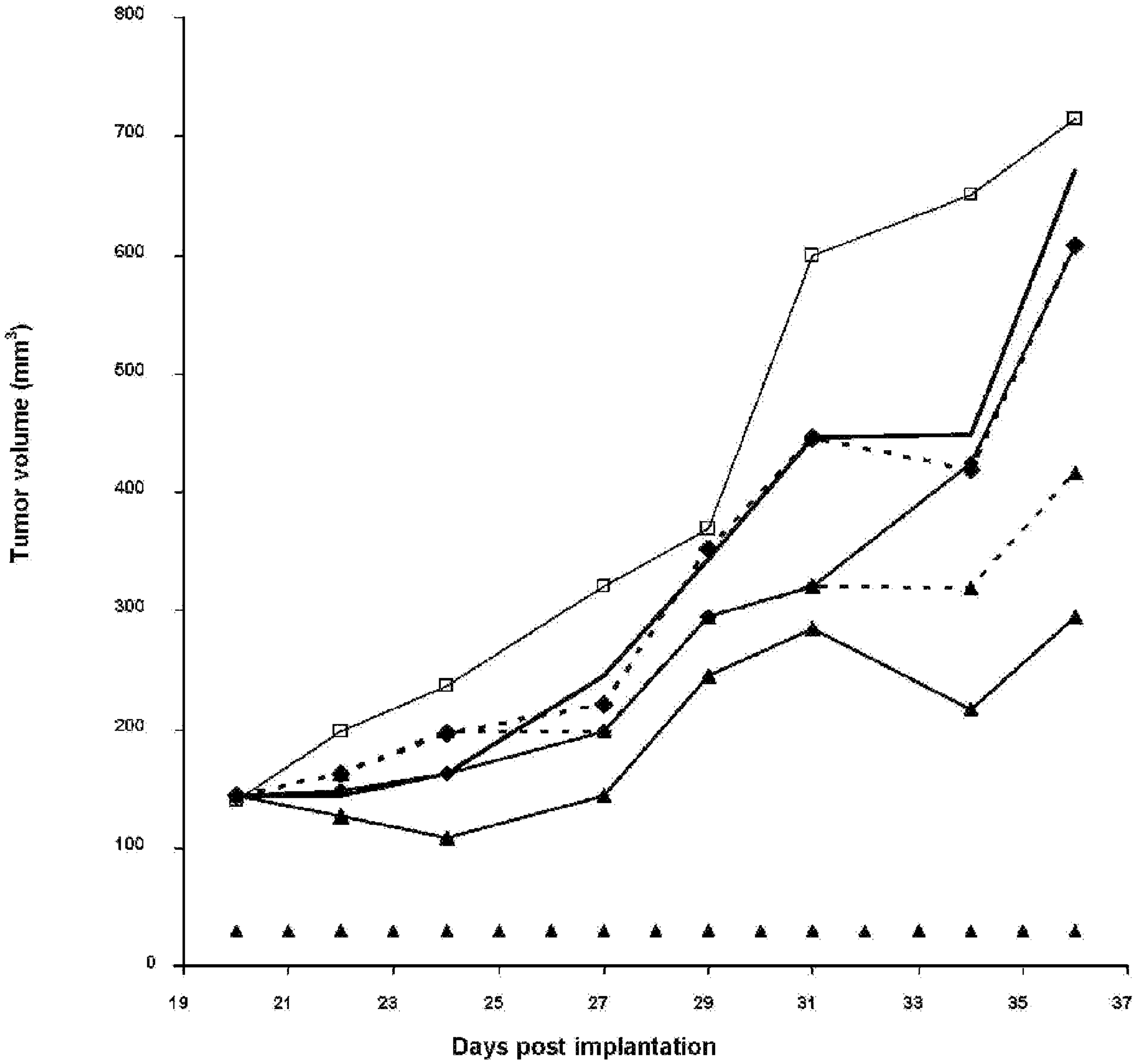

800
700
600
500
400
300
200
100
0
Tumor volume (mm3)
19
21
23
25
27
29
31
33
35
37
Days post implantation


FIG.14

Europäisches Patentamt
European Patent Office
Office européen des brevets

# EUROPEAN SEARCH REPORT

Application Number
EP 11 30 6172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | S. A. AZIZ ET AL: "Vertical Targeting of the Phosphatidylinositol-3 Kinase Pathway as a Strategy for Treating Melanoma", CLINICAL CANCER RESEARCH, vol. 16, no. 24, 15 December 2010 (2010-12-15), pages 6029-6039, XP55013948, ISSN: 1078-0432, DOI: 10.1158/1078-0432.CCR-10-1490 * page 6033, column 1 - column 2 * * page 6038, column 2, paragraph 2 * ----- | 1-37 | INV. A61K31/4184 A61K31/437 A61K31/5377 A61K45/06 A61P35/00 |
| Y | FR 2 951 169 A1 (SANOFI AVENTIS [FR]) 15 April 2011 (2011-04-15) * the whole document * ----- | 1-37 | |
| Y | JIERU MENG ET AL: "Combination Treatment with MEK and AKT Inhibitors Is More Effective than Each Drug Alone in Human Non-Small Cell Lung Cancer In Vitro and In Vivo", PLOS ONE, vol. 5, no. 11, 1 January 2010 (2010-01-01), page E14124, XP55013949, ISSN: 1932-6203, DOI: 10.1371/journal.pone.0014124 * abstract * * page 1, column 1 * * page 8, column 1 - column 2 * ----- | 1-37 | TECHNICAL FIELDS SEARCHED (IPC) A61K |
| | -/-- | | |

1

The present search report has been drawn up for all claims

| Place of search | Date of completion of the search | Examiner |
|---|---|---|
| Munich | 6 December 2011 | Uryga-Polowy, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 30 6172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Y. N. V. GOPAL ET AL: "Basal and Treatment-Induced Activation of AKT Mediates Resistance to Cell Death by AZD6244 (ARRY-142886) in Braf-Mutant Human Cutaneous Melanoma Cells", CANCER RESEARCH, vol. 70, no. 21, 1 November 2010 (2010-11-01), pages 8736-8747, XP55013950, ISSN: 0008-5472, DOI: 10.1158/0008-5472.CAN-10-0902 * abstract * * page 8741, column 2 - page 8742, column 2 * ----- | 1-37 | |
| Y | HUNG HUYNH: "AZD6244 (ARRY-142886) enhances the antitumor activity of rapamycin in mouse models of human hepatocellular carcinoma", CANCER, vol. 116, no. 5, 1 March 2010 (2010-03-01), pages 1315-1325, XP55013951, ISSN: 0008-543X, DOI: 10.1002/cncr.24863 * abstract * * page 1315 * * page 1318 * * page 1319; table 1 * * page 1323, column 1 - column 2 * ----- | 1-37 | |

TECHNICAL FIELDS SEARCHED (IPC)

The present search report has been drawn up for all claims

| Place of search | Date of completion of the search | Examiner |
|---|---|---|
| Munich | 6 December 2011 | Uryga-Polowy, V |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 30 6172

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2011

| Patent document cited in search report | Publication date | Patent family member(s) | Publication date |
|---|---|---|---|
| FR 2951169 A1 | 15-04-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003077914 A
- WO 2007002325 A
- WO 2011001114 A


### Non-patent literature cited in the description


- **D. B. SOLIT et al.** *Nature,* 2006, vol. 439, 358-362
- Remington's Pharmaceutical Sciences. Mack Publishing Company, 1985
- **S. M. BERGE et al.** Pharmaceutical Salts. *J. Pharm. Sci.,* 1977, vol. 66, 1-19
- Remington's Pharmaceutical Sciences. Mack Publishing Company, 1990
- **R.STRAETEMANS.** *Biometrical Journal,* 2005, 47